# EUROPEAN PATENT SPECIFICATION

(11) **EP 3 355 902 B1**
(45) Date of publication and mention of the grant of the patent: **13.04.2022**
(21) Application number: 16779272.0
(22) Date of filing: 27.09.2016
(51) Int. Cl.: A61K 31/439, A61K 31/4545, A61P 35/00

(54) **COMBINATION OF A PD-1 AXIS BINDING ANTAGONIST AND AN ALK INHIBITOR FOR TREATING ALK-NEGATIVE CANCER**
KOMBINATION EINES PD-1-ACHSEN-BINDUNGSANTAGONISTEN UND EINES ALK-INHIBITORS ZUR BEHANDLUNG VON ALK-NEGATIVEM KREBS
COMBINAISON D'UN ANTAGONISTE DE LA LIAISON DE L'AXE PD-1 ET D'UN INHIBITEUR DE ALK DANS LE TRAITEMENT DU CANCER ALK-NÉGATIF

(30) Priority: 30.09.2015 US 201562235406 P; 09.09.2016 US 201662385430 P; 12.09.2016 US 201662393609 P
(43) Date of publication of application: 08.08.2018
(73) Proprietor: Merck Patent GmbH, 64293 Darmstadt (DE); Pfizer Inc., New York, NY 10017 (US)
(72) Inventor: LIN, Chia-Yang, Palo Alto, California 94303 (US); PRIOR, Wei Wei Wu, Redwood City, CA 94065 (US); QU, Yan, Half Moon Bay, California 94019 (US); WANG, Changyu, Union City, California 94587 (US)
(74) Representative: Seemann, Joachim
(86) International application number: PCT/US2016/053939
(87) International publication number: WO 2017/058780

(56) References cited:
- BIN-CHIN LIAO, ET AL: "Trating patients with ALK-positiv non-small cell lung cancer: latest evidence and management strategy", THERAPEUTICS ADVANCES IN MEDICAL ONCOLOGY, vol. 7, no. 5, 1 September 2015 (2015-09-01), pages 274-290, XP002765122,
- Anonymous: "NCT02511184 on 2015_09_21: ClinicalTrials.gov Archive", , 21 September 2015 (2015-09-21), XP055328030, Retrieved from the Internet: URL:https://clinicaltrials.gov/archive/NCT 02511184/2015_09_21 [retrieved on 2016-12-12]
- BERGETHON K ET AL: "ROS1 rearrangements define a unique molecular class of lung cancers", JOURNAL OF CLINICAL ONCOLOGY, AMERICAN SOCIETY OF CLINICAL ONCOLOGY, US, vol. 30, no. 8, 10 March 2012 (2012-03-10) , pages 863-870, XP009165222, ISSN: 0732-183X, DOI: 10.1200/JCO.2011.35.6345

## Description

This application claims the benefit of U. S. Provisional Application No. 62/235,406 filed September 30, 2015, U. S. Provisional Application No. 62/385,430 filed September 9, 2016, and U.S. Provisional Application No. 62/393,609 filed September 12, 2016.

### FIELD OF THE INVENTION

The present invention relates to an anti-PD-L1 antibody for use in combination and the ALK inhibitor crizotinib for treating ALK-negative cancer.

### BACKGROUND OF THE INVENTION

The mechanism of co-stimulation of T-cells has gained significant therapeutic interest in recent years for its potential to enhance cell-based immune response. Costimulatory molecules expressed on antigen-presenting cells (APCs) promote and induce T-cells to promote clonal expansion, cytokine secretion and effector function. In the absence of co-stimulation, T-cells can become refractory to antigen stimulation, do not mount an effective immune response, and further may result in exhaustion or tolerance to foreign antigens. Lenschow et al., Ann. Rev. Immunol. 14:233 (1996). Recently, it has been discovered that T cell dysfunction or anergy occurs concurrently with an induced and sustained expression of the inhibitory receptor, programmed death 1 polypeptide (PD-1). As a result, therapeutic targeting of PD-1 and other molecules which signal through interactions with PD-1, such as programmed death ligand 1 (PD-L1) and programmed death ligand 2 (PD-L2) are an area of intense interest.

PD-L1 is overexpressed in many cancers and is often associated with poor prognosis (Okazaki T et al., Intern. Immun. 2007 19(7):813) (Thompson RH et al., Cancer Res 2006, 66(7):3381). Interestingly, the majority of tumor infiltrating T lymphocytes predominantly express PD-1 , in contrast to T lymphocytes in normal tissues and peripheral blood T lymphocytes indicating that up-regulation of PD-1 on tumor-reactive T cells can contribute to impaired antitumor immune responses (Ahmadzadeh et al,Blood 2009 1 14(8): 1537). This may be due to exploitation of PD-L1 signaling mediated by PD-L1 expressing tumor cells interacting with PD-1 expressing T cells to result in attenuation of T cell activation and evasion of immune surveillance (Sharpe et al., Nat Rev 2002) (Keir ME et al., 2008 Annu. Rev. Immunol. 26:677). Therefore, inhibition of the PD-L1 /PD-1 interaction may enhance CD8+ T cell-mediated killing of tumors.

The inhibition of PD-1 axis signaling through its direct ligands (e.g., PD-L1, PD-L2) has been proposed as a means to enhance T cell immunity for the treatment of cancer (e.g., tumor immunity). Moreover, similar enhancements to T cell immunity have been observed by inhibiting the binding of PD-L1 to the binding partner B7-1. Furthermore, combining inhibition of PD-1 signaling with other pathways would further optimize therapeutic properties

Anaplastic lymphoma kinase (ALK) is a member of the receptor tyrosine kinase superfamily, and at an amino acid sequence level is most closely related to members such as c-ros oncogene 1 (ROS1), leukocyte tyrosine kinase (LTK), the insulin receptor and c-Met (hepatic growth factor receptor) (Kostich M et al, Genome Biology 2002, 3, 1-12). ALK is largely expressed in the developing nervous system (Iwahara T et al, Oncogene 1997, 14, 439-449). Its relative abundance does tend to decrease in the adult animal, though its expression is maintained in certain regions of the brain, spinal cord and the eye (Vernersson et al., Gene Expression Patterns 2006, 6, 448-461).

ALK also has an important role in oncology (Webb TR et al, Expert Reviews in Anticancer Therapy 2009 9 331-355). Point mutations in the full length ALK enzyme that lead to activation of the enzyme, and also increase in expression of the full length enzyme, have both been shown to lead to neuroblastoma (Ogawa S et al., Cancer Sci 2011 102:302-308). In addition, the fusion of ALK with other proteins due to genetic translocation events has also been shown to lead to activated kinase domain associated with cancer. A number of such ALK translocations leading to gene fusions are seen in lymphomas, the most prevalent being the nucleophosmin (NPM)-ALK fusion seen in anaplastic large cell lymphomas (ALCL). ALK fusion with EML4 leads to a chimeric protein (EML4-ALK) responsible for 2-7% of non-small cell lung carcinomas (NSCLC) (Soda M et al, Nature 2007 448 561-567).

ALK inhibitors have been approved for the treatment of ALK-positive metastatic non-small cell lung cancer (NSCLC). However, since the EML4-ALK fusion kinase is aberrantly activated in only a small population of NSCLC carcinomas, there remains a need for optimal therapy for treating, stabilizing, preventing, and/or delaying development of various cancers in ALK-negative cancers. An optimal therapeutic treatment would provide combine blockade of PD-1 receptor/ligand interaction with an ALK inhibitor to bring further therapeutic benefit to patients suffering from cancers including both ALK-positive and ALK-negative cancers.

In addition to their direct activity on ALK-positive tumor cells, ALK inhibitors may affect the signaling and interaction of immune cells, including T cells and dendritic cells (DC). Without wishing to be bound by theory, it is believed that ALK inhibitors may exert immunomodulatory effects either directly or indirectly, e.g., by inhibition of wild-type ALK expressed on immune cells, or by stimulating or inhibiting the effects of downstream signaling pathways in immune cells. Alternately, it is possible that wild-type ALk activity is associated with immune suppressive mechanisms mediated by non-immune cells such as tumor cells and/or stromal cells. Thus, the combination of an ALK inhibitor and a PD-1 axis binding antagonist may provide beneficial effects even in ALK-negative cancers.

Liao et al., Ther. Adv. Med. Onc. 7(5), 275-290, discloses that crizotinib is effective in treating ALK-negative NSCLC, while clinicaltrials.gov entry NCT02511184 discloses a clinical trial of crizotinib in combination with an anti-PD-L1 antibody in the treatment of ALK-positive advanced NSCLC.

### SUMMARY OF THE INVENTION

The present invention is defined by the appended claims. The present invention describes an anti-PD-L1 antibody for use in combination and the ALK inhibitor crizotinib in the treatment of ALK-negative cancers.

The ALK-negative cancers in particular do not have c-Met amplification and/or ROS1 fusion and/or have no known alterations in either c-Met or ROS1. For example, in some embodiments the ALK-negative cancer will also have no known c-Met or ROS1 alterations, which have been predicted to exhibit sensitivity to an ALK inhibitor such as crizotinib alone. Zou HY et al. (2007, May 1) Cancer Res. 1;67(0):4408-17; Shaw AT et al. (2014, Nov 20). N.Engl.J.Med.; 371(21): 1963-71.

The ALK-negative cancer may be any cancer that does not test positive for an activating ALK aberration such as an ALK fusion event or an oncogenic ALK mutation, including EML4-ALK, KIF5B-ALK, TFG-ALK, KLC1-ALK, NPM-ALK, TMP3-ALK, TPM4-ALK, ATIC-ALK or CLTC-ALK. An individual who is diagnosed with an ALK-negative cancer will have a cancer which tests negative for any of the above-identified oncogenic mutations, but may otherwise express wild-type ALK. The ALK-negative cancer may be a solid tumor. In some embodiments of the above uses of the invention, the cancer is a solid tumor and in some embodiments, the ALK-negative solid tumor is a melanoma, colon cancer, bladder cancer, breast cancer, clear cell kidney cancer, head/neck squamous cell carcinoma, rectal cancer, lung squamous cell carcinoma, thyroid cancer, bladder cancer, cervical cancer, uterine cancer, endometrial cancer, lung adenocarcinoma, ovarian cancer, papillary kidney cancer, non-small-cell lung cancer (NSCLC), ovarian cancer, pancreatic cancer, prostate cancer, renal cell cancer, small-cell lung cancer (SCLC) and triple negative breast cancer. In some embodiments, the ALK-negative cancer may be at early stage or at late stage, and/or is an advanced or metastatic solid tumor malignancy. In some embodiments, the individual treated is a human.

Also provided herein is an anti-PD-L1 antibody for use in combination and the ALK inhibitor crizotinib of enhancing immune function in an individual having an ALK-negative cancer comprising administering an effective amount of a PD-1 axis binding antagonist and an ALK inhibitor.

In some embodiments, the PD-L 1 binding antagonist is an antibody (e.g., antibody MPDL3280A, MDX- 1105, MEDI4736 or MSB0010718C described herein).

In some embodiments, the ALK inhibitor is administered sequentially (at separate times) or concurrently (during the same time) relative to the anti-PD-L1 antibody. In some embodiments, the ALK inhibitor is administered either continuously or intermittently. In some embodiments, the ALK inhibitor is administered before administration of the anti-PD-L1 antibody, simultaneously with administration of the anti-PD-L1 antibody, or after administration of the PD-1 axis binding antagonist. In some embodiments, the ALK inhibitor and the anti-PD-L1 antibody are administered with different dosing frequency.

In some embodiments, selecting a dosage regimen (also referred to herein as an administration regimen) for a combination regime of the invention depends on several factors, including the serum or tissue turnover rate of the entity, the level of symptoms, the immunogenicity of the entity, and the accessibility of the target cells, tissue or organ in the individual being treated. Preferably, a dosage regimen maximizes the amount of each therapeutic agent delivered to the patient consistent with an acceptable level of side effects. Accordingly, the dose amount and dosing frequency of each biotherapeutic and chemotherapeutic agent in the combination depends in part on the particular therapeutic agent, the severity of the cancer being treated, and patient characteristics. Guidance in selecting appropriate doses of antibodies, cytokines, and small molecules are available. *See, e.g.,* Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, NY; Bach (ed.) (1993) Monoclonal Antibodies and Peptide Therapy in Autoimmune Diseases, Marcel Dekker, New York, NY; Baert et al. (2003) New Engl. J. Med. 348:601-608; Milgrom et al. (1999) New Engl. J. Med. 341:1966-1973; Slamon et al. (2001) New Engl. J. Med. 344:783-792; Beniaminovitz et al. (2000) New Engl. J. Med. 342:613-619; Ghosh et al. (2003) New Engl. J. Med. 348:24-32; Lipsky et al. (2000) New Engl. J. Med. 343:1594-1602; Physicians' Desk Reference 2003 (Physicians' Desk Reference, 57th Ed); Medical Economics Company; ISBN: 1563634457; 57th edition (November 2002). Determination of the appropriate dosage regimen may be made by the clinician, *e.g.,* using parameters or factors known or suspected in the art to affect treatment or predicted to affect treatment, and will depend, for example, the patient's clinical history (e.g., previous therapy), the type and stage of the cancer to be treated and biomarkers of response to one or more of the therapeutic agents in the combination therapy.

Biotherapeutic agents in a combination regime of the invention may be administered by continuous infusion, or by doses at intervals of, *e.g.,* daily, every other day, three times per week, or one time each week, two weeks, three weeks, monthly, bimonthly, etc. A total weekly dose is generally at least about 0.05 µg/kg, 0.2 µg/kg, 0.5 µg/kg, 1 µg/kg, 10 µg/kg, 100 µg/kg, 0.2 mg/kg, 1.0 mg/kg, 2.0 mg/kg, 10 mg/kg, 25 mg/kg, 50 mg/kg body weight or more. See, *e.g.,* Yang et al. (2003) New Engl. J. Med. 349:427-434; Herold et al. (2002) New Engl. J. Med. 346:1692-1698; Liu et al. (1999) J. Neurol. Neurosurg. Psych. 67:451-456; Portielji et al. (20003) Cancer Immunol. Immunother. 52:133-144.

Dosage units for an anti-PD-L1 antibody may be expressed as a flat dose, i.e., 100 mg, 200 mg, 300 mg, or as a patient-specific dose, i.e., mg/kg (mg therapeutic agent/kg of body weight) or mg/m² (quantity in milligrams per square meter of body surface area).

In certain embodiments, a subject will be administered an intravenous (IV) infusion of a medicament comprising any of the anti-PD-L1 antibodies described herein.

In one embodiment of the invention, the anti-PD-L1 antibody in the combination therapy is avelumab (MSB0010718C), which is administered intravenously or in a liquid dosage form at a dose selected from the group consisting of any one of : 1 mg/kg Q2W, 2 mg/kg Q2W, 3 mg/kg Q2W, 5 mg/kg Q2W, 10 mg Q2W, 1 mg/kg Q3W, 2 mg/kg Q3W, 3 mg/kg Q3W, 5 mg/kg Q3W, and 10 mg Q3W.

The optimal dose for an anti-PD-L1 antibody in combination with crizotinib may be identified by dose escalation of one or both of these agents. The crizotinib may be administered orally (PO), either once daily (QD) or twice daily (BID), with or without food on a continuous schedule starting on Cycle 1 Day 1. A anti-PD-L1 antibody such as avelumab may be administered as a 30-minute to 1-hr intravenous (IV) infusion every 2 weeks (Q2W), every 3 weeks (Q3W) or in case of dose reduction, every 4 weeks (Q4W), starting on Cycle 1 Day 1, except in the case of the crizotinib lead-in. On the day of ALK inhibitor administration, the crizotinib may be given prior to or after administration of the anti-PD-L1 antibody.

In another embodiment, crizotinib can be administered at 250 mg BID, 200 mg BID, or 250 mg QD, and the anti-PD-L1 antibodyis administered at a starting dose of 2 mg/kg, or 5 mg/kg or 10 mg/kg, at a dosing interval of Q2W, Q3W or alternately Q4W In one embodiment, the crizotinib is administered at 250 mg BID for a 3-week lead-in period and then the anti-PD-L1 antibody is administered at a starting dose of 2 mg/kg Q3W or 200 mg Q3W after the lead-in period. In another embodiment, the crizotinib is administered at 250 mg BID and the anti-PD-L1 antibodyis administered at a starting dose of 2 mg/kg Q4W. In another embodiment, the crizotinib is administered at 200 mg BID and the anti-PD-L1 antibodyis administered at a starting dose of 2 mg/kg Q3W. In another embodiment, the crizotinib is administered at 200 mg BID and the anti-PD-L1 antibodyis administered at a starting dose of 2 mg/kg Q4W In another embodiment, the crizotinib is administered at 250 mg QD and the anti-PD-L1 antibody is administered at a starting dose of 2 mg/kg Q4W.

In some embodiments, the patient is treated with a 3-week lead-in period of single-agent crizotinib directly preceding the combination administration of the crizotinib and anti-PD-L1 antibody.

In some embodiments, a treatment cycle begins with the first day of combination treatment and last for 3 weeks. In such embodiments, the combination therapy is preferably administered for at least 18 weeks (6 cycles of treatment), more preferably at least 24 weeks (8 cycles of treatment), and even more preferably at least 2 weeks after the patient achieves a CR.

In some embodiments, the dose of crizotinib is increased up to a maximum dose of 250 mg BID if the patient tolerates the combination treatment at a lower total dose of crizotinib.

### I. Definitions

"ALK" means anaplastic lymphoma kinase receptor tyrosine kinase. All references to ALK herein will be understood to include references to both ALK and to oncogenic variants thereof, including ALK fusions (including without limitation EML4-ALK, KIF5B-ALK, TFG-ALK, KLC1-ALK and NPM-ALK) and selected oncogenic mutations of ALK.

"ALK inhibitor" means an inhibitor of anaplastic lymphoma kinase (ALK) and/or its oncogenic variants, i.e., ALK fusions and selected oncogenic mutations of ALK. Frequently, the inhibitor is a small molecule inhibitor of ALK. ALK inhibitors may also provide inhibition activitity against c-Met, ROS1, RON, Trk, LTK and/or the insulin receptor.

"ALK-negative cancer" means any cancer which tests negative for an activating ALK aberration, such as an ALK fusion event or oncogenic ALK mutation, including EML4-ALK, KIF5B-ALK, TFG-ALK, KLC1-ALK, NPM-ALK, TMP3-ALK, TPM4-ALK, ATIC-ALK or CLTC-ALK. An individual who is diagnosed with or identified as having an ALK-negative cancer will have a cancer which tests negative for activating ALK aberrations, but may otherwise express wild-type ALK. In some embodiments, wild-type ALK is expressed in the tumor microenvironment, or in any of the immune cells including T cells, B cells, NK cells, dendritic cells, macrophages, myeloid-derived suppressor cells and/or in tumor and/or stromal cells.

In some embodiments, an individual who is diagnosed with or identified as having an ALK-negative cancer may furthermore express any one of or a combination of wild-type c-Met, ROS1, RON, LTK, Trk (TrkA, TrkB, TrkC), and/or insulin receptor in the tumor microenvironment, or in any of the immune cells including T cells, B cells, NK cells, dendritic cells, macrophages, myeloid-derived suppressor cells and/or in tumor and/or stromal cells.

A variety of methods for the detection of ALK aberrations have been described. (Shackelford RE et al., Genes Cancer, 2014, 5(1-2): 1-14). In some embodiments, status of the ALK gene is assessed using an FDA approved test. In some embodiments, the status of the ALK gene is detected using an assay that analyzes ALK or ALK fusion polynucleotides, such as the Vysis ALK Break Apart FISH Probe Kit (available from Abbott Molecular), or such as assays employing RT-PCR or Next Generation sequencing (NGS) technology. In other embodiments, the status of the ALK gene is inferred based on ALK expression, which is detected using a diagnostic anti-ALK antibody, or antigen binding fragment thereof, in an IHC assay on an FFPE or frozen tissue section of a tumor sample removed from a patient afflicted with cancer.

Crizotinib is the compound, 3-[(1R)-1-(2,6-dichloro-3-fluorophenyl)ethoxy]-5-(1-piperidin-4-ylpyrazol-4-yl)pyridin-2-amine, which is known as PF-02341066, having the following structure: 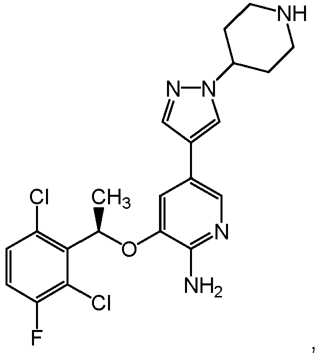 or a pharmaceutically acceptable salt thereof.

Crizotinib is an inhibitor of anaplastic lymphoma kinase (ALK) and its oncogenic variants (i.e., ALK fusion events and selected oncogenic ALK mutations), as well as the hepatocyte growth factor receptor (HGFR, c-Met), c-ros oncogene 1 (ROS1) and its oncogenic variants, Recepteur d'Origine Nantais (RON) receptor tyrosine kinases (RTKs), LTK, Trk (TrkA, TrkB, TrkC), and/or insulin receptor

Xalkori^{®} (crizotinib) has been approved in the United States for the treatment of patients with metastatic non-small cell lung cancer (NSCLC) whose tumors are anaplastic lymphoma kinase (ALK)-positive as detected by an FDA-approved test, and has also been approved for the treatment of ALK-positive NSCLC in Europe, Japan and other jurisdictions.

Crizotinib, as well as pharmaceutically acceptable salts thereof, is described in International Publication Nos. WO 2006/021884, WO 2006/021881 and WO 2007/066185, and in U.S. Patent Nos. 7,858,643, 8,217,057 and 8,785,632. The use of crizotinib in treating abnormal cell growth, such as cancers, mediated by ALK or c-MET/HGFR is described in WO 2007/06617 and U.S. Patent No. 7,825,137. The use of crizotinib in treating ROS mediated cancers is described in WO2013/017989.

| | |
|---|---|
| SEQ ID NO: 17 | EVQLLESGGGLVQPGGSLRLSCAASGFTFSSYIMWVRQAPGKGL EWVSSIYPSGGITFYADKGRFTISRDNSKNTLYLQMNSLRAEDTA VYYCARIKLGTVTTVDYWGQ GTLVTVSS |
| MSB0010718C heavy chain | |
| From WO13079174 | |
| SEQ ID NO: 18 | QSALTQPASVSGSPGQSITISCTGTSSDVGGYNYVSWYQQHPGK APKLMIYDVSNRPSGVSNRFSGSKSGNTASLTISGLQAEDEADYY CSSYTSSSTRVFGTGTKVTVL |
| MSB0010718C light chain | |
| From WO13079174 | |

The term "dysfunction" in the context of immune dysfunction, refers to a state of reduced immune responsiveness to antigenic stimulation. The term includes the common elements of both exhaustion and/or anergy in which antigen recognition may occur, but the ensuing immune response is ineffective to control infection or tumor growth.

The term "dysfunctional", as used herein, also includes refractory or unresponsive to antigen recognition, specifically, impaired capacity to translate antigen recognition into downstream T-cell effector functions, such as proliferation, cytokine production (e.g., IL-2) and/or target cell killing.

The term "anergy" refers to the state of unresponsiveness to antigen stimulation resulting from incomplete or insufficient signals delivered through the T-cell receptor (e.g. increase in intracellular Ca⁺² in the absence of ras-activation). T cell anergy can also result upon stimulation with antigen in the absence of co-stimulation, resulting in the cell becoming refractory to subsequent activation by the antigen even in the context of costimulation. The unresponsive state can often be overriden by the presence of Interleukin-2. Anergic T-cells do not undergo clonal expansion and/or acquire effector functions.

The term "exhaustion" refers to T cell exhaustion as a state of T cell dysfunction that arises from sustained TCR signaling that occurs during many chronic infections and cancer. It is distinguished from anergy in that it arises not through incomplete or deficient signaling, but from sustained signaling. It is defined by poor effector function, sustained expression of inhibitory receptors and a transcriptional state distinct from that of functional effector or memory T cells. Exhaustion prevents optimal control of infection and tumors. Exhaustion can result from both extrinsic negative regulatory pathways (e.g., immunoregulatory cytokines) as well as cell intrinsic negative regulatory (costimulatory) pathways (PD-1 , B7-H3, B7-H4, etc.).

"Enhancing T-cell function" means to induce, cause or stimulate a T-cell to have a sustained or amplified biological function, or renew or reactivate exhausted or inactive T-cells. Examples of enhancing T-cell function include: increased secretion of γ-interferon from CD8⁺ T-cells, increased proliferation, increased antigen responsiveness (e.g. , viral, pathogen, or tumor clearance) relative to such levels before the intervention. In one embodiment, the level of. enhancement is as least 50%, alternatively 60%, 70%, 80%, 90%, 100%, 1 20%, 150%, 200%. The manner of measuring this enhancement is known to one of ordinary skill in the art..

A "T cell dysfunctional disorder" is a disorder or condition of T-cells characterized by decreased responsiveness to antigenic stimulation. In a particular embodiment, a T-cell dysfunctional disorder is a disorder that is specifically associated with inappropriate increased signaling through PD-1 . In another embodiment, a T-cell dysfunctional disorder is one in which T-cells are anergic or have decreased ability to secrete cytokines, proliferate, or execute cytolytic activity. In a specific aspect, the decreased responsiveness results in ineffective control of a pathogen or tumor expressing an immunogen. Examples of T have upcell dysfunctional disorders characterized by T-cell dysfunction include unresolved acute infection, chronic infection and tumor immunity.

"Tumor immunity" refers to the process in which tumors evade immune recognition and clearance. Thus, as a therapeutic concept, tumor immunity is "treated" when such evasion is attenuated, and the tumors are recognized and attacked by the immune system. Examples of tumor recognition include tumor binding, tumor shrinkage and tumor clearance.

"Immunogenecity" refers to the ability of a particular substance to provoke an immune response. Tumors are immunogenic and enhancing tumor immunogenicity aids in the clearance of the tumor cells by the immune response. Examples of enhancing tumor immunogenicity include treatment with anti-PDL antibodies and an ALK inhibitor.

"Sustained response" refers to the sustained effect on reducing tumor growth after cessation of a treatment. For example, the tumor size may remain to be the same or smaller as compared to the size at the beginning of the administration phase. In some embodiments, the sustained response has a duration at least the same as the treatment duration, at least 1.5X, 2.0X, 2.5X, or 3.0X length of the treatment duration.

The term "antibody " includes monoclonal antibodies (including full length antibodies which have an immunoglobulin Fc region), antibody compositions with polyepitopic specificity, multispecific antibodies (e.g. , bispecific antibodies, diabodies, and single-chain molecules, as well as antibody fragments (e.g., Fab, F(ab')2, and Fv). The term "immunoglobulin" (Ig) is used interchangeably with "antibody" herein.

The basic 4-chain antibody unit is a heterotetrameric glycoprotein composed of two identical light (L) chains and two identical heavy (H) chains. An IgM antibody consists of 5 of the basic heterotetramer units along with an additional polypeptide called a J chain, and contains 10 antigen binding sites, while IgA antibodies comprise from 2-5 of the basic 4-chain units which can polymerize to form polyvalent assemblages in combination with the J chain. In the case of IgGs, the 4-chain unit is generally about 150,000 daltons. Each L chain is linked to an H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain (V_{H}) followed by three constant domains (CH) for each of the a and γ chains and four CH domains for µ and ε isotypes. Each L chain has at the N-terminus, a variable domain (VL) followed by a constant domain at its other end. The VL is aligned with the V_{H} and the CL is aligned with the first constant domain of the heavy chain (CH I ). Particular amino acid residues are believed to form an interface between the light chain and heavy chain variable domains. The pairing of a VH and VL together forms a single antigen-binding site. For the structure and properties of the different classes of antibodies, see e.g. , Basic and Clinical Immunology, 8th Edition, Daniel P. Sties, Abba I. Terr and Tristram G. Parsolw (eds), Appleton & Lange, Norwalk, CT, 1994, page 7 1 and Chapter 6. The L chain from any vertebrate species can be assigned to one of two clearly distinct types, called kappa and lambda, based on the amino acid sequences of their constant domains. Depending on the amino acid sequence of the constant domain of their heavy chains (CH), immunoglobulins can be assigned to different classes or isotypes. There are five classes of immunoglobulins: IgA, IgD, IgE, IgG and IgM, having heavy chains designated α, δ, ε, γ and µ, respectively. The γ and a classes are further divided into subclasses on the basis of relatively minor differences in the CH sequence and function, e.g. , humans express the following subclasses: IgG1 , IgG2A, IgG2B, IgG3, IgG4, IgA1 and IgA2.

The "variable region" or "variable domain" of an antibody refers to the amino-terminal domains of the heavy or light chain of the antibody. The variable domains of the heavy chain and light chain may be referred to as " VH" and " VL", respectively. These domains are generally the most variable parts of the antibody (relative to other antibodies of the same class) and contain the antigen binding sites.

The term "variable" refers to the fact that certain segments of the variable domains differ extensively in sequence among antibodies. The V domain mediates antigen binding and defines the specificity of a particular antibody for its particular antigen. However, the variability is not evenly distributed across the entire span of the variable domains. Instead, it is concentrated in three segments called hypervariable regions (HVRs) both in the light-chain and the heavy chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen binding site of antibodies (see Kabat et ai, Sequences of Immunological Interest, Fifth Edition, National Institute of Health, Bethesda, MD ( 1991 )). The constant domains are not involved directly in the binding of antibody to an antigen, but exhibit various effector functions, such as participation of the antibody in antibody-dependent cellular toxicity.

The term "monoclonal antibody " as used herein refers to an antibody obtained from a population of substantially homogeneous antibodies, i.e., the individual antibodies comprising the population are identical except for possible naturally occurring mutations and/or post-translation modifications (e.g. , isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g. , Kohler and Milstein. , Nature, 256:495-97 ( 1975); Hongo et al, Hybridoma, 14 (3): 253-260 ( 1995), Harlow et al , Antibodies: A Laboratory Manual, (Cold Spring Harbor Laboratory Press, 2nd ed. 1988); Hammerling et al , in: Monoclonal Antibodies and T-Cell Hybridomas 563-681 (Elsevier, N.Y., 1981)), recombinant DNA methods (see, e.g. , U.S. Patent No. 4,816,567), phage-display technologies (see, e.g. , Clackson et al, Nature, 352: 624-628 ( 1991 ); Marks et al, J. Mol. Biol. 222: 581 -597 ( 1992); Sidhu et al, J. Mol. Biol 338(2): 299-310 (2004); Lee et al. , J. Mol. Biol. 340(5): 1073-1093 (2004); Fellouse, Proc. Natl. Acad. Sci. USA 101 (34): 12467-12472 (2004); and Lee et al, J. Immunol. Methods 284(1 -2): 1 19- 132 (2004), and technologies for producing human or human-like antibodies in animals that have parts or all of the human immunoglobulin loci or genes encoding human immunoglobulin sequences (see, e.g. , WO 1998/24893; WO1996/34096; WO 1996/33735 ; WO 1991 /10741 ; Jakobovits et al , Proc. Natl. Acad. Sci. USA 90: 255 1 ( 1993); Jakobovits et al, Nature 362: 255-258 ( 1993); Bruggemann et al, Year in Immunol. 7:33 ( 1993); U.S. Patent Nos. 5,545,807; 5,545,806; 5,569,825 ; 5,625, 126; 5,633,425 ; and 5,661 ,016; Marks et al, Bio/Technology 10: 779-783 ( 1992); Lonberg et al, Nature 368: 856-859 (1994); Morrison, Nature 368: 812-813 ( 1994); Fishwild et al , Nature Biotechnol. 14: 845-851 ( 1996); Neuberger, Nature Biotechnol. 14: 826 ( 1996); and Lonberg and Huszar, Intern. Rev. Immunol. 13 : 65-93 ( 1995).

The term "naked antibody" refers to an antibody that is not conjugated to a cytotoxic moiety or radiolabel.

The terms "full-length antibody," "intact antibody" or "whole antibody" are used interchangeably to refer to an antibody in its substantially intact form, as opposed to an antibody fragment. Specifically whole antibodies include those with heavy and light chains including an Fc region. The constant domains may be native sequence constant domains (e.g. , human native sequence constant domains) or amino acid sequence variants thereof. In some cases, the intact antibody may have one or more effector functions.

An "antibody fragment" comprises a portion of an intact antibody, preferably the antigen binding and/or the variable region of the intact antibody. Examples of antibody fragments include Fab, Fab', F(ab')₂ and Fv fragments; diabodies; linear antibodies (see U.S. Patent 5,641 ,870, Example 2; Zapata et al, Protein Eng. 8(10): 1057-1062 [1995]); single-chain antibody molecules and multispecific antibodies formed from antibody fragments. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (Cn I ). Each Fab fragment is monovalent with respect to antigen binding, i.e., it has a single antigen-binding site. Pepsin treatment of an antibody yields a single large F(ab')₂ fragment which roughly corresponds to two disulfide linked Fab fragments having different antigen-binding activity and is still capable of cross-linking antigen. Fab' fragments differ from Fab fragments by having a few additional residues at the carboxy terminus of the CH I domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The Fc fragment comprises the carboxy-terminal portions of both H chains held together by disulfides. The effector functions of antibodies are determined by sequences in the Fc region, the region which is also recognized by Fc receptors (FcR) found on certain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site.

"Single-chain Fv " also abbreviated as "sFv " or "scFv " are antibody fragments that comprise the VH and VL antibody domains connected into a single polypeptide chain. Preferably, the sFv polypeptide further comprises a polypeptide linker between the VH and V_{L} domains which enables the sFv to form the desired structure for antigen binding. For a review of the sFv, see Pluckthun in The Pharmacology of Monoclonal Antibodies, vol. 1 13, Rosenburg and Moore eds., Springer-Verlag, New York, pp. 269-315 (1994).

"Functional fragments" of the antibodies of the invention comprise a portion of an intact antibody, generally including the antigen binding or variable region of the intact antibody or the Fc region of an antibody which retains or has modified FcR binding capability. Examples of antibody fragments include linear antibody, single-chain antibody molecules and multispecific antibodies formed from antibody fragments.

The term "diabodies" refers to small antibody fragments prepared by constructing sFv fragments (see preceding paragraph) with short linkers (about 5- 10) residues) between the V_{H} and VL domains such that inter-chain but not intra-chain pairing of the V domains is achieved, thereby resulting in a bivalent fragment, i.e. , a fragment having two antigen-binding sites. Bispecific diabodies are heterodimers of two "crossover" sFv fragments in which the V_{H} and V_{L} domains of the two antibodies are present on different polypeptide chains. Diabodies are described in greater detail in, for example, EP 404,097; WO 93/1 1 161 ; Hollinger et ai, Proc. Natl. Acad. Sci. USA 90: 6444-6448 ( 1993).

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et ai, Proc. Natl. Acad. Sci. USA, 81:6851 -6855 ( 1984)). Chimeric antibodies of interest herein include PRIMATIZED^{®} antibodies wherein the antigen-binding region of the antibody is derived from an antibody produced by, e.g. , immunizing macaque monkeys with an antigen of interest. As used herein, "humanized antibody" is used a subset of "chimeric antibodies."

"Humanized" forms of non-human {e.g., murine) antibodies are chimeric antibodies that contain minimal sequence derived from non-human immunoglobulin. In one embodiment, a humanized antibody is a human immunoglobulin (recipient antibody) in which residues from an HVR (hereinafter defined) of the recipient are replaced by residues from an HVR of a non-human species (donor antibody) such as mouse, rat, rabbit or non-human primate having the desired specificity, affinity, and/or capacity. In some instances, framework ("FR") residues of the human immunoglobulin are replaced by corresponding non-human residues. Furthermore, humanized antibodies may comprise residues that are not found in the recipient antibody or in the donor antibody. These modifications may be made to further refine antibody performance, such as binding affinity. In general, a humanized antibody will comprise substantially all of at least one, and typically two, variable domains, in which all or substantially all of the hypervariable loops correspond to those of a non-human immunoglobulin sequence, and all or substantially all of the FR regions are those of a human immunoglobulin sequence, although the FR regions may include one or more individual FR residue substitutions that improve antibody performance, such as binding affinity, isomerization, immunogenicity, etc. The number of these amino acid substitutions in the FR are typically no more than 6 in the H chain, and in the L chain, no'more than 3. The humanized antibody optionally will also comprise at least a portion of an immunoglobulin constant region (Fc), typically that of a human immunoglobulin. For further details, see, e.g. , Jones et ai. Nature 321 :522-525 (1986); Riechmann et al, Nature 332:323-329 (1988); and Presta, Curr. Op. Struct. Biol. 2:593-596 (1992). See also, for example, Vaswani and Hamilton, Ann. Allergy, Asthma & Immunol. 1 : 105- 1 15 (1998); Harris, Biochem. Soc. Transactions 23: 1035-1038 (1995); Hurle and Gross, Curr. Op. Biotech. 5:428-433 (1994); and U.S. Pat. Nos. 6,982,321 and 7,087,409.

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991 ); Marks el al. , J. Mol. Biol., 222:581 (1991). Also available for the preparation of human monoclonal antibodies are methods described in Cole et ai, Monoclonal Antibodies and Cancer Therapy, Alan R. Liss, p. 77 (1985); Boerner et al. , J. Immunol. , 147(I):86-95 (1991). See also van Dijk and van de Winkel, Curr. Opin. Pharmacol, 5: 368-74 (2001). Human antibodies can be prepared by administering the antigen to a transgenic animal that has been modified to produce such antibodies in response to antigenic challenge, but whose endogenous loci have been disabled, e.g., immunized xenomice (see, e.g., U.S. Pat. Nos. 6,075, 181 and 6, 150,584 regarding XENOMOUSE^{™} technology). See also, for example, Li et al, Proc. Natl. Acad. Sci. USA, 103 :3557-3562 (2006) regarding human antibodies generated via a human B-cell hybridoma technology.

The term "hypervariable region," "HVR," or "HV" when used herein refers to the regions of an antibody variable domain which are hypervariable in sequence and/or form structurally defined loops. Generally, antibodies comprise six HVRs; three in the VH (H1, H2, H3), and three in the VL (L1 , L2, L3). In native antibodies, H3 and L3 display the most diversity of the six HVRs, and H3 in particular is believed to play a unique role in conferring fine specificity to antibodies. See, e.g., Xu et ai, Immunity J_3:37-45 (2000); Johnson and Wu, in Methods in Molecular Biology 248: 1 -25 (Lo, ed., Human Press, Totowa, NJ, 2003). Indeed, naturally occurring camelid antibodies consisting of a heavy chain only are functional and stable in the absence of light chain. See, e.g., Hamers-Casterman et ai, Nature 363:446-448 (1993); Sheriff ef al, Nature Struct. Biol 3:733-736 (1996).

A number of HVR delineations are in use and are encompassed herein. The Kabat Complementarity Determining Regions (CDRs) are based on sequence variability and are the most commonly used (Kabat et al. Sequences of Proteins of Immunological Interest, 5th Ed. Public Health Service, National Institutes of Health, Bethesda, MD. (1991 )). Chothia refers instead to the location of the structural loops (Chothia and Lesk, J. Mol. Biol. 196:901 -917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91 -L96 | L89-L96 |
| H1 | H31 -H35B | H26-H35B | H26-H32 | H30-H35B (Kabat numbering) |
| H1 | H31 -H35 | H26-H35 | H26-H32 | H30-H35 (Chothia numbering) |
| H2 | H50-H65 | H50-H58 | H53-H55 | H47-H58 |
| H3 | H95-H 102 | H95-H 102 | H96-H101 | H93-H101 |

HVRs may comprise "extended HVRs" as follows: 24-36 or 24-34 (L1 ), 46-56 or 50-56 (L2) and 89-97 or 89-96 (L3) in the VL and 26-35 (H1), 50-65 or 49-65 (H2) and 93- 102, 94-102, or 95- 102 (H3) in the VH. The variable domain residues are numbered according to Kabat et al., supra, for each of these definitions.

The expression "variable-domain residue-numbering as in Kabat" or "amino-acid-position numbering as in Kabat," and variations thereof, refers to the numbering system used for heavy-chain variable domains or light-chain variable domains of the compilation of antibodies in Kabat et al., supra. Using this numbering system, the actual linear amino acid sequence may contain fewer or additional amino acids corresponding to a shortening of, or insertion into, a FR or HVR of the variable domain. For example, a heavy-chain variable domain may include a single amino acid insert (residue 52a according to Kabat) after residue 52 of H2 and inserted residues (e.g. residues 82a, 82b, and 82c, etc. according to Kabat) after heavy-chain FR residue 82. The Kabat numbering of residues may be determined for a given antibody by alignment at regions of homology of the sequence of the antibody with a "standard" Kabat numbered sequence.

"Framework" or "FR" residues are those variable-domain residues other than the HVR residues as herein defined.

A "human consensus framework or "acceptor human framework" is a framework that represents the most commonly occurring amino acid residues in a selection of human immunoglobulin VL or VH framework sequences. Generally, the selection of human immunoglobulin VL or VH sequences is from a subgroup of variable domain sequences.

Generally, the subgroup of sequences is a subgroup as in Kabat et ai , Sequences of Proteins of Immunological Interest, 5lh Ed. Public Health Service, National Institutes of Health, Bethesda, MD (1991 ). Examples include for the VL, the subgroup may be subgroup kappa I, kappa II, kappa III or kappa IV as in Kabat et al, supra. Additionally, for the VH, the subgroup may be subgroup I, subgroup II, or subgroup III as in Kabat et al., supra. Alternatively, a human consensus framework can be derived from the above in which particular residues, such as when a human framework residue is selected based on its homology to the donor framework by aligning the donor framework sequence with a collection of various human framework sequences. An acceptor human framework "derived from" a human immunoglobulin framework or a human consensus framework may comprise the same amino acid sequence thereof, or it may contain pre-existing amino acid sequence changes. In some embodiments, the number of pre-existing amino acid changes are 10 or less, 9 or less, 8 or less, 7 or less, 6 or less, 5 or less, 4 or less, 3 or less, or 2 or less.

An "amino-acid modification" at a specified position, e.g. of the Fc region, refers to the substitution or deletion of the specified residue, or the insertion of at least one amino acid residue adjacent the specified residue. Insertion "adjacent" to a specified residue means insertion within one to two residues thereof. The insertion may be N-terminal or C-terminal to the specified residue. The preferred amino acid modification herein is a substitution.

An "affinity-matured" antibody is one with one or more alterations in one or more HVRs thereof that result in an improvement in the affinity of the antibody for antigen, compared to a parent antibody that does not possess those alteration(s). In one embodiment, an affinity-matured antibody has nanomolar or even picomolar affinities for the target antigen. Affinity-matured antibodies are produced by procedures known in the art. For example, Marks et al., Bio/Technology 10:779-783 ( 1992) describes affinity maturation by VH- and VL-domain shuffling. Random mutagenesis of HVR and/or framework residues is described by, for example: Barbas et al. Proc Nat. Acad. Sci. USA 91 :3809-3813 ( 1994); Schier et al. Gene 169: 147- 155 (1995); Yelton et al. J. Immunol. 155: 1994-2004 ( 1995); Jackson et al , J. Immunol. 154(7):33 10-9 (1995); and Hawkins et al, J. Mol. Biol. 226:889-896 ( 1992).

As use herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In one embodiment, the extent of binding of an antibody to an unrelated target is less than about 10% of the binding of the antibody to the target as measured, e.g., by a radioimmunoassay (RIA). In certain embodiments, an antibody that specifically binds to a target has a dissociation constant (Kd) of < 1 µM), < 100 n , < 10 nM, < 1 nM, or < 0.1 nM. In certain embodiments, an antibody specifically binds to an epitope on a protein that is conserved among the protein from different species. In another embodiment, specific binding can include, but does not require exclusive binding.

A "fusion protein" and a "fusion polypeptide" refer to a polypeptide having two portions covalently linked together, where each of the portions is a polypeptide having a different property. The property may be a biological property, such as activity in vitro or in vivo. The property may also be simple chemical or physical property, such as binding to a target molecule, catalysis of a reaction, etc. The two portions may be linked directly by a single peptide bond or through a peptide linker but are in reading frame with each other.

A "blocking" antibody or an "antagonist" antibody is one that inhibits or reduces a biological activity of the antigen it binds. In some embodiments, blocking antibodies or antagonist antibodies substantially or completely inhibit the biological activity of the antigen. The anti-PD-L1 antibodies of the invention block the signaling through PD-1 so as to restore a functional response by T-cells (e.g., proliferation, cytokine production, target cell killing) from a dysfunctional state to antigen stimulation.

An "agonist" or activating antibody is one that enhances or initiates signaling by the antigen to which it binds. In some embodiments, agonist antibodies cause or activate signaling without the presence of the natural ligand.

The term "Fc region" herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. Suitable native-sequence Fc regions for use in the antibodies of the invention include human IgG I , IgG2 (IgG2A, IgG2B), IgG3 and IgG4.

"Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcyRI, FcyRII, and FeyRIII subclasses, including allelic variants and alternatively spliced forms of these receptors, FcyRII receptors include FcyRIIA (an "activating receptor") and FcyRIIB (an "inhibiting receptor"), which have similar amino acid sequences that differ primarily in the cytoplasmic domains thereof. Activating receptor FcyRIIA contains an immunoreceptor tyrosine-based activation motif (ITAM) in its cytoplasmic domain. Inhibiting receptor FcyRIIB contains an immunoreceptor tyrosine-based inhibition motif (ITΓM) in its cytoplasmic domain, (see M. Daeron, Annu. Rev. Immunol. J_5 :203-234 ( 1997). FcRs are reviewed in Ravetch and inet, Annu. Rev. Immunol. 9: 457-92 ( 1991 ); Capel et al. , Immunomethods 4: 25-34 ( 1994); and de Haas et al., J. Lab. Clin. Med. 126: 330-41 ( 1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus. Guyer et ai, J. Immunol. 1 17: 587 (1976) and Kim et ai, J. Immunol. 24: 249 ( 1994). Methods of measuring binding to FcRn are known (see, e.g. , Ghetie and Ward, Immunol. Today 1 8: (12): 592-8 ( 1997); Ghetie et ai, Nature Biotechnology 15 (7): 637-40 ( 1997); Hinton et ai, J. Biol. Chem. 279 (8): 6213-6 (2004); WO 2004/92219 (Hinton et ai). Binding to FcRn in vivo and serum half-life of human FcRn high-affinity binding polypeptides can be assayed, e.g., in transgenic mice or transfected human cell lines expressing human FcRn, or in primates to which the polypeptides having a variant Fc region are administered. WO 2004/42072 (Presta) describes antibody variants which improved or diminished binding to FcRs. See also, e.g. , Shields et ai , J. Biol. Chem. 9(2): 6591 - 6604 (2001).

The phrase "substantially reduced," or "substantially different," as used herein, denotes a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (e.g. , Kd values). The difference between said two values is, for example, greater than about 10%, greater than about 20%, greater than about 30%, greater than about 40%, and/or greater than about 50% as a function of the value for the reference/comparator molecule.

The term "substantially similar" or "substantially the same," as used herein, denotes a sufficiently high degree of similarity between two numeric values (for example, one associated with an antibody of the invention and the other associated with a reference/comparator antibody), such that one of skill in the art would consider the difference between the two values to be of little or no biological and/or statistical significance within the context of the biological characteristic measured by said values (e.g. , Kd values). The difference between said two values is, for example, less than about 50%, less than about 40%, less than about 30%, less than about 20%, and/or less than about 10% as a function of the reference/comparator value.

"Carriers" as used herein include pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, polyethylene glycol (PEG), and PLURONICS^{™}.

As used herein, the term "treatment" refers to clinical intervention designed to alter the natural course of the individual or cell being treated during the course of clinical pathology. Desirable effects of treatment include decreasing the rate of disease progression, ameliorating or palliating the disease state, and remission or improved prognosis. For example, an individual is successfully "treated" if one or more symptoms associated with cancer are mitigated or eliminated, includingreducing the proliferation of (or destroying) cancerous cells, decreasing symptoms resulting from the disease, increasing the quality of life of those suffering from the disease, decreasing the dose of other medications required to treat the disease, delaying the progression of the disease, and/or prolonging survival of individuals. As used herein, "delaying progression of a disease" means to defer, hinder, slow, retard, stabilize, and/or postpone development of the disease (such as cancer). This delay can be of varying lengths of time, depending on the history of the disease and/or individual being treated. As is evident to one skilled in the art, a sufficient or significant delay can, in effect, encompass prevention, in that the individual does not develop the disease. For example, a late stage cancer, such as development of metastasis, may be delayed.

An "effective amount" is at least the minimum concentration required to effect a measurable improvement or prevention of a particular disorder. An effective amount herein may vary according to factors such as the disease state, age, sex, and weight of the patient, and the ability of the antibody to elicit a desired response in the individual. An effective amount is also one in which any toxic or detrimental effects of the treatment are outweighed by the therapeutically beneficial effects. For prophylactic use, beneficial or desired results include results such as eliminating or reducing the risk, lessening the severity, or delaying the onset of the disease, including biochemical, histological and/or behavioral symptoms of the disease, its complications and intermediate pathological phenotypes presenting during development of the disease. For therapeutic use, beneficial or desired results include clinical results such as decreasing one or more symptoms resulting from the disease, increasing the quality of life of those suffering from the disease, decreasing the dose of other medications required to treat the disease, enhancing effect of another medication such as via targeting, delaying the progression of the disease, and/or prolonging survival. In the case of cancer or tumor, an effective amount of the drug may have the effect in reducing the number of cancer cells; reducing the tumor size; inhibiting (i.e., slow to some extent or desirably stop) cancer cell infiltration into peripheral organs; inhibit (i.e., slow to some extent and desirably stop) tumor metastasis; inhibiting to some extent tumor growth; and/or relieving to some extent one or more of the symptoms associated with the disorder. An effective amount can be administered in one or more administrations. For purposes of this invention, an effective amount of drug, compound, or pharmaceutical composition is an amount sufficient to accomplish prophylactic or therapeutic treatment either directly or indirectly. As is understood in the clinical context, an effective amount of a drug, compound, or pharmaceutical composition may or may not be achieved in conjunction with another drug, compound, or pharmaceutical composition. Thus, an "effective amount" may be considered in the context of administering one or more therapeutic agents, and a single agent may be considered to be given in an effective amount if, in conjunction with one or more other agents, a desirable result may be or is achieved.

As used herein, "in combination with" or "in conjunction with" refers to administration of one treatment modality in addition to another treatment modality. As such, "in combination with" or "in conjunction with" refers to administration of one treatment modality before, during, or after administration of the other treatment modality to the individual.

As used herein, "complete response" or "CR" refers to disappearance of all target lesions; "partial response" or "PR" refers to at least a 30% decrease in the sum of the longest diameters (SLD) of target lesions, taking as reference the baseline SLD; and "stable disease" or "SD" refers to neither sufficient shrinkage of target lesions to qualify for PR, nor sufficient increase to qualify for PD, taking as reference the smallest SLD since the treatment started.

As used herein, "progressive disease" or "PD" refers to at least a 20% increase in the SLD of target lesions, taking as reference the smallest SLD recorded since the treatment started or the presence of one or more new lesions.

As used herein, "progression free survival" (PFS) refers to the length of time during and after treatment during which the disease being treated (e.g., cancer) does not get worse. Progression-free survival may include the amount of time patients have experienced a complete response or a partial response, as well as the amount of time patients have experienced stable disease.

As used herein, "overall response rate" (ORR) refers to the sum of complete response (CR) rate and partial response (PR) rate.

As used herein, "overall survival" refers to the percentage of individuals in a group who are likely to be alive after a particular duration of time.

A "chemotherapeutic agent" is a chemical compound useful in the treatment of cancer. Examples of chemotherapeutic agents include alkylating agents such as thiotepa and cyclophosphamide (CYTOXAN^{®}); alkyl sulfonates such as busulfan, improsulfan, and piposulfan; aziridines such as.benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL^{®}); beta-lapachone; lapachol; colchicines; betulinic acid; a camptothecin (including the synthetic analogue topotecan (HYCAMTIN^{®}), CPT- 11 (irinotecan, CAMPTOSAR^{®}), acetylcamptothecin, scopolectin, and 9-aminocamptothecin); bryostatin; pemetrexed; callystatin; CC- 1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB 1 -TM1 ); eleutherobin; pancratistatin; TLK-286; CDP323, an oral alpha-4 integrin inhibitor; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g. , calicheamicin, especially calicheamicin gamma II and calicheamicin omegal I (see, e.g., Nicolaou et ai, Angew. Chem Intl. Ed. Engl., 33 : 183-186 ( 1994)); dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including ADRIAMYCIN^{®}, morpholino-doxorubicin, cyanomorpholino-doxorubicin, 2-pyrrolino-doxorubicin, doxorubicin HC1 liposome injection (DOXIL^{®}) and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate, gemcitabine (GEMZAR^{®}), tegafur (UFTORAL^{®}), capecitabine (XELODA^{®}), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, and imatinib (a 2-phenylaminopyrimidine derivative), as well as other c- it inhibitors; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDIS1NE^{®}, FILDESIN^{®}); dacarbazine; mannomustine; mitobronitol; mitolactol; pipobroman; gacytosine; arabinoside ("Ara-C"); thiotepa; taxoids, e.g., paclitaxel (TAXOL^{®}), albumin-engineered nanoparticle formulation of paclitaxel (ABRAXANE^{™}), and doxetaxel (TAXOTERE^{®}); chloranbucil; 6-thioguanine; mercaptopurine; methotrexate; platinum analogs such as cisplatin and carboplatin; vinblastine (VELBAN^{®}); platinum; etoposide (VP-16); ifosfamide; mitoxantrone; vincristine (ONCOVIN^{®}); oxaliplatin; leucovovin; vinorelbine (NAVELBINE^{®}); novantrone; edatrexate; daunomycin; aminopterin; ibandronate; topoisomerase inhibitor RFS 2000; difluorometlhylomithine (DMFO); retinoids such as retinoic acid; pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN^{™}) combined with 5-FU and leucovovin.

Additional examples of chemotherapeutic agents include anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX^{®} tamoxifen), raloxifene (EVISTA^{®}), droloxifene, 4-hydroxytamoxifen, trioxifene, keoxifene, LY 1 1 7018, onapristone, and toremifene (FARESTON^{®}); anti-progesterones; estrogen receptor down-regulators (ERDs); estrogen receptor antagonists such as fulvestrant (FASLODEX^{®}); agents that function to suppress or shut down the ovaries, for example, leutinizing hormone-releasing hormone (LHRFI) agonists such as leuprolide acetate (LUPRON^{®} and ELIGARD^{®}), goserelin acetate, buserelin acetate and tripterelin; anti-androgens such as fiutamide, nilutamide and bicalutamide; and aromatase inhibitors that inhibit the enzyme aromatase, which regulates estrogen production in the adrenal glands, such as, for example, 4(5)-imidazoles, aminoglutethimide, megestrol acetate (MEGASE^{®}), exemestane (AROMASIN^{®}), formestanie, fadrozole, vorozole (RJVISOR^{®}), letrozole (FEMARA^{®}), and anastrozole (ARIMIDEX^{®}). In addition, such definition of chemotherapeutic agents includes bisphosphonates such as clodronate (for example, BONEFOS^{®} or OSTAC^{®}), etidronate (DIDROCAL^{®}), NE-58095, zoledronic acid/zoledronate (ZOMETA^{®}), alendronate (FOSAMAX^{®}), pamidronate (AREDIA^{®}), tiludronate (SKELID^{®}), or risedronate (ACTONEL^{®}); as well as troxacitabine (a 1 ,3-dioxolane nucleoside cytosine analog); anti-sense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE^{®} vaccine and gene therapy vaccines, for example, ALLOVECTlN^{®} vaccine, LEUVECTlN^{®} vaccine, and VAXID^{®} vaccine; topoisomerase 1 inhibitor (e.g. , LURTOTECAN^{®}); an anti-estrogen such as fulvestrant; a Kit inhibitor such as imatinib or EXEL-0862 (a tyrosine kinase inhibitor); EGFR inhibitor such as erlotinib or cetuximab; an anti-VEGF inhibitor such as bevacizumab; arinotecan; rmRH (e.g., ABARELIX^{®}); lapatinib and lapatinib ditosylate (an ErbB-2 and EGFR dual tyrosine kinase small-molecule inhibitor also known as GW572016); 17AAG (geldanamycin derivative that is a heat shock protein (Hsp) 90 poison), and pharmaceutically acceptable salts, acids or derivatives of any of the above.

As used herein, the term "cytokine" refers generically to proteins released by one cell population that act on another cell as intercellular mediators or have an autocrine effect on the cells producing the proteins. Examples of such cytokines include lymphokines, monokines; interleukins ("ILs") such as IL- 1 , IL- Ia, IL-2, IL-3, IL-4, IL-5, IL-6, IL-7, IL-8, IL-9, IL10, IL-1 1 , IL-12, IL-13, IL-15, IL-17A-F, IL-18 to IL-29 (such as IL-23), IL-31 , including PROLEUKIN^{®} rIL-2; a tumor-necrosis factor such as TNF-a or TNF-β, TGF- I -3; and other polypeptide factors including leukemia inhibitory factor ("LIF"), ciliary neurotrophic factor ("CNTF"), CNTF-like cytokine ("CLC"), cardiotrophin ("CT"), and kit ligand (" L").

As used herein, the term "chemokine" refers to soluble factors (e.g., cytokines) that have the ability to selectively induce chemotaxis and activation of leukocytes. They also trigger processes of angiogenesis, inflammation, wound healing, and tumorigenesis. Example chemokines include IL-8, a human homolog of murine keratinocyte chemoattractant (KC).

As used herein and in the appended claims, the singular forms "a," "or," and "the" include plural referents unless the context clearly dictates otherwise.

Reference to "about" a value or parameter herein includes (and describes) variations that are directed to that value or parameter per se. For example, description referring to "about X" includes description of "X".

A pharmaceutically acceptable salt may involve the inclusion of another molecule such as an acetate ion, a succinate ion or other counter ion. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

If the compound of the invention is a base, the desired pharmaceutically acceptable salt may be prepared by any suitable method available in the art, for example, treatment of the free base with an inorganic acid, such as hydrochloric acid, hydrobromic acid, sulfuric acid, nitric acid, methanesulfonic acid, phosphoric acid, or with an organic acid, such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid.

If the compound of the invention is an acid, the desired pharmaceutically acceptable salt may be prepared by any suitable method, for example, treatment of the free acid with an inorganic or organic base, such as an amine (primary, secondary or tertiary), an alkali metal hydroxide or alkaline earth metal hydroxide.

Illustrative examples of suitable salts include organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum and lithium.

The phrase "pharmaceutically acceptable" indicates that the substance or composition must be compatible chemically and/or toxicologically, with the other ingredients comprising a formulation, and/or the mammal being treated therewith.

A "solvate" refers to an association or complex of one or more solvent molecules and a compound of the invention. Examples of solvents that form solvates include water, isopropanol, ethanol, methanol, DMSO, ethyl acetate, acetic acid and ethanolamine. The term "hydrate" refers to the complex where the solvent molecule is water.

### II. Anti PD-L1 Antibodies

In some embodiments, the anti-PD-L1 antibody is capable of inhibiting binding between PD-L1 and PD-1 and/or between PD-L1 and B7- 1. In some embodiments, the anti-PD-L1 antibody is a monoclonal antibody. In some embodiments, the anti-PD-L1 antibody is an antibody fragment selected from the group consisting of Fab, Fab'-SH, Fv, scFv, and (Fab')2 fragments. In some embodiments, the anti-PD-L1 antibody is a humanized antibody. In some embodiments, the anti-PD-L1 antibody is a human antibody. In some embodiments, the anti-PD-L1 antibody is selected from the group consisting of MPDL3280A (YW243.55.S70 or atezolizumab), MDX-1105, MEDI4736 and MSB0010718C. In some embodiments, the anti-PD-L1 antibody is MPDL3280 (from clone YW243.55.S70, heavy and light chain variable region sequences shown in SEQ ID Nos. 14and 15, respectively), an anti-PD-L1 antibody described in WO 2010/077634 A1. In other embodiments, the anti-PD-L1 antibody is MDX-1105, also known as BMS-936559, an anti-PD-L1 antibody described in WO2007/005874. In yet other embodiments, the anti-PD-L1 antibody is MEDI4736, an anti-PD-L1 described in WO2011/066389. In other embodiments, the anti-PD-L1 antibody is MSB0010718C (heavy and light chain variable sequences as shown in SEQ ID NO: 17 and 18, respectively) is an anti-PD-L1 described in WO13079174.

The anti-PD-L1 antibodies useful in this invention, including compositions containing such antibodies, such as YW243.55.S70 as described in WO 2010/077634, may be used in combination with crizotinib to treat an ALK-negative cancer.

In a still further specific aspect, the anti-PD-L1 antibody further comprises a human or murine constant region. In a still further aspect, the human constant region is selected from the group consisting of IgGI , IgG2, IgG2, IgG3, IgG4. In a still further specific aspect, the human constant region is IgGI . In a still further aspect, the murine constant region is selected from the group consisting of IgG I , IgG2A, IgG2B, IgG3. In a still further aspect, the murine constant region if IgG2A. In a still further specific aspect, the antibody has reduced or minimal effector function. In a still further specific aspect, the minimal effector function results from production in prokaryotic cells. In a still further specific aspect the minimal effector function results from an "effector-less Fc mutation" or aglycosylation. In still a further aspect, the effector-less Fc mutation is an N297A or D265A N297A substitution in the constant region.

The antibody or antigen binding fragment thereof, may be made using methods known in the art, for example, by a process comprising culturing a host cell containing nucleic acid encoding any of the previously described anti-PD-L1 antibodies or antigen-binding fragment in a form suitable for expression, under conditions suitable to produce such antibody or fragment, and recovering the antibody or fragment.

In a still further embodiment, the invention provides for a composition comprising an anti-PD-L1 antibody or antigen binding fragment thereof as provided herein and at least one pharmaceutically acceptable carrier. In some embodiments, the anti-PD-L1 antibody or antigen binding fragment thereof administered to the individual is a composition comprising one or more pharmaceutically acceptable carrier. Any of the pharmaceutically acceptable carrier described herein or known in the art may be used.

### III. Crizotinib

The crizotinib administered may be in a pharmaceutical composition or formulation. In some embodiments, the pharmaceutical composition or formulation comprises crizotinib described herein and a pharmaceutically acceptable carrier or excipient.

### EXAMPLES

### Example 1: ALK inhibitor impact on T cell proliferation and viability

To assess the relative impact of ALK inhibitors, PF-06463922 and PF-02341066 (crizotinib, Xalkori^{®}, Pfizer Inc) on CD3/CD28-stimulated effector cells, activation assays were performed which compared the ALK inhibitors to tyrosine kinase inhibitors, sunitinib and axitinib (Sutent^{®} and Inlyta^{®}, Pfizer Inc). In published studies, administration of sunitinib and axitinib resulted in dose-dependent inhibition of both cell proliferation and cell viability but with axitinib showing only minor toxicity effects in peripheral blood mononuclear cells (PMBCs). Stehle F. et al. J Biol Chem. 2013 Jun 7;288 (23): 16334-4.

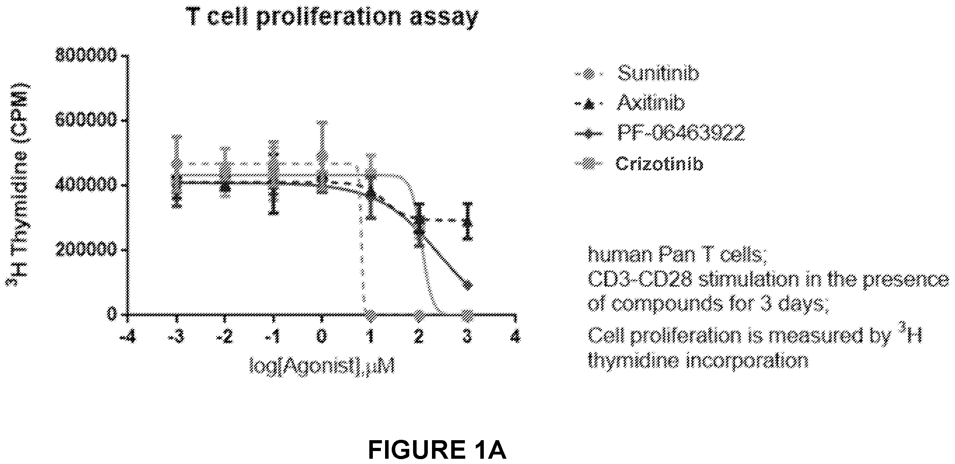

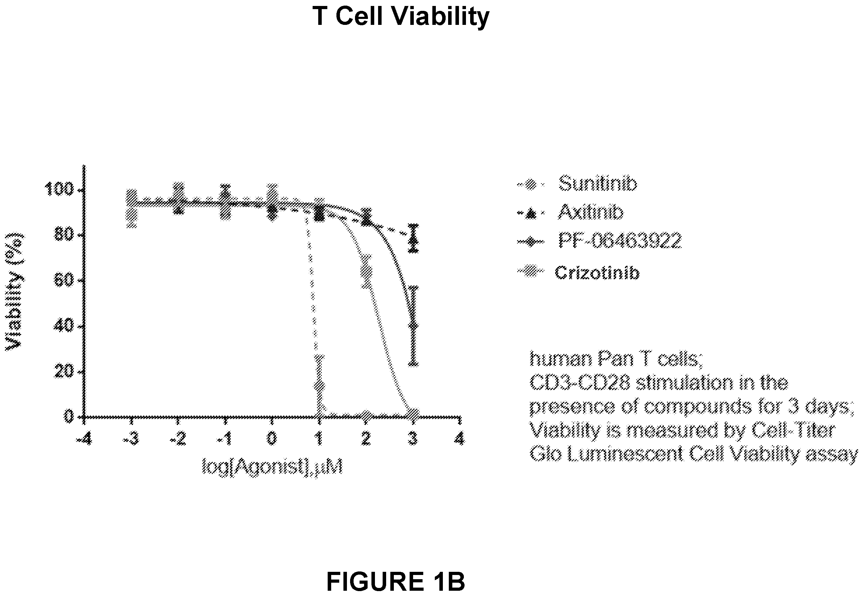

Briefly, human cells were isolated from healthy donors' PBMCs by using EasySep^{™} Human T Cell Enrichment Kit (Stemcell Technologies) according to manufacturer's instruction. ALK inhibitors PF-06463922 and crizotinib were titrated such that the final culture concentration was 0.1 % DMSO in a total volume of 150 µ per well. Cells were cultured in RPMI 1640 medium supplemented with 10% FCS and 100 I.U./mL penicillin/streptomycin in humidified incubator at 37 °C with 5% CO2. Post isolation, cells were directly stimulated with Dynabeads^{®} Human T-Activator CD3 or CD3/CD28 (Life Technologies) in the presence of each compound at indicated concentration ranges, .001, .01, 0.1, 1 , 10 µg/ml concentration, that cover clinically effective concentration for three days: (PF-06463922 at 100 nM, crizotinib at 80 nM, sunitinib at 12-20 nM, axitinib at 0.2-0.6 nM or DMSO vehicle). Culture supernatants were collected on Day 2 or 3 for cytokine analysis. Plates were then harvested on DNA specific filter papers (Perkin Elmer) using Harvester96 (Tomtec Life Sciences). The radiolabeled filters were covered with beta scintillation liquid (Perkin Elmer) and read in Microbeta^{®} counter plates (Perkin Elmer). ³H Thymidine was pulsed on Day 2 for T cell proliferation assay. The impact by sunitinib and axitinib on T cell proliferation and viability were repeated as previously published (Figures 1A and 1B). Moreover, treatment of activated T cells with ALK inhibitors PF-06463922 and crizotinib did not have adverse effects on T cell proliferation, with dosage levels that exceeded sunitinib and more closely approximated axitinib (Figure 1A).

Table 3 below provides the thymidine incorporation (CPM) values for each of the respective inhibitors.

**Table 3: Thymidine incorporation(CPM)**

| log[Agonist], µM | Sunitinib | Axitinib | PF-06463922 | crizotinib |
|---|---|---|---|---|
| | Mean | Mean | Mean | Mean |
| 0 | 493570.3 ±59347.45 | 437978 ±5214.787 | 399866.7 ±10135.82 | 432360.3 ±29488.63 |
| -3 | 469071 ±48070.7 | 394950 ±19054.05 | 401448.3 ±37205.07 | 421221.3 ±24333.6 |
| -2 | 444679.3 ±13911.18 | 408592.3 ±11690 | 411422.3 ±13377.95 | 442995.3 ±42508.08 |
| -1 | 468847.7 ±39073.88 | 407235 ±52888.48 | 416579.3 ±23523.21 | 438641 ±47103.88 |
| 1 | 332 ±105.9733 | 385672.3 ±9004.015 | 364978.7 ±37421.4 | 436099.3 ±34171.33 |
| 2 | 117.3333 ±16.23097 | 300379.3 ±25594 | 258875.3 ±25269.1 | 270672.7 ±18298.53 |
| 3 | 171 ±25 | 291128.7 ±31076.09 | 94120.66 ±2728.719 | 437.6667 ±104.9704 |

Cell viability was measured by Cell-Titer Glo Luminescent Cell Viability assay^{®} (Promega, Madison). Figure 1B indicates that PF-06463922 and crizotinib did not result in dose-dependent decreases in T cell viability, with dosage levels that again exceeded sunitinib and more closely approximated axitinib. By analogy, PF-06463922 and crizotinib appears suitable for implementation in dosing regimens which incorporate immunotherapy. Table 4 below provides the Luciferase RLU values reflecting T cell viability for each of the respective inhibitors.

**Table 4: Luciferase RLU**

| log[Agonist], µM | Sunitinib | Axitinib | PF-06463922 | crizotinib |
|---|---|---|---|---|
| | Mean | Mean | Mean | Mean |
| 0 | 98.24227 ±2.168515 | 92.62644 ±0.522128 | 88.53202 ±0.756657 | 94.22272 ±1.813741 |
| -3 | 96.57127 ±1.710999 | 97.36216 ±1.185823 | 98.16747 ±0.660638 | 89.07632 ±2.842621 |
| -2 | 98.03569 ±2.63407 | 95.54089 ±3.149818 | 95.86486 ±0.620189 | 93.67803 ±1.564283 |
| -1 | 91.57024 ±2.195313 | 91.35086 ±1.526172 | 97.8625 ±2.287887 | 95.6927 ±1.826977 |
| 1 | 13.95176 ±7.493037 | 89.78527 ±1.428145 | 89.118 ±1.283329 | 91.71838 ±2.35425 |
| 2 | 0.850193 ±0.259612 | 86.62844 ±0.877947 | 88.6231 ±1.572783 | 64.23526 ±3.748341 |
| 3 | 1.826584 0.58776 | 78.81053 ±3.223431 | 40.36231 ±9.73577 | 1.47364 ±0.16934 |

### Example 2: ALK inhibition enhanced anti-tumor activity of anti-PD-L1 antibodies in ALK-negative colorectal and melanoma tumors in vivo

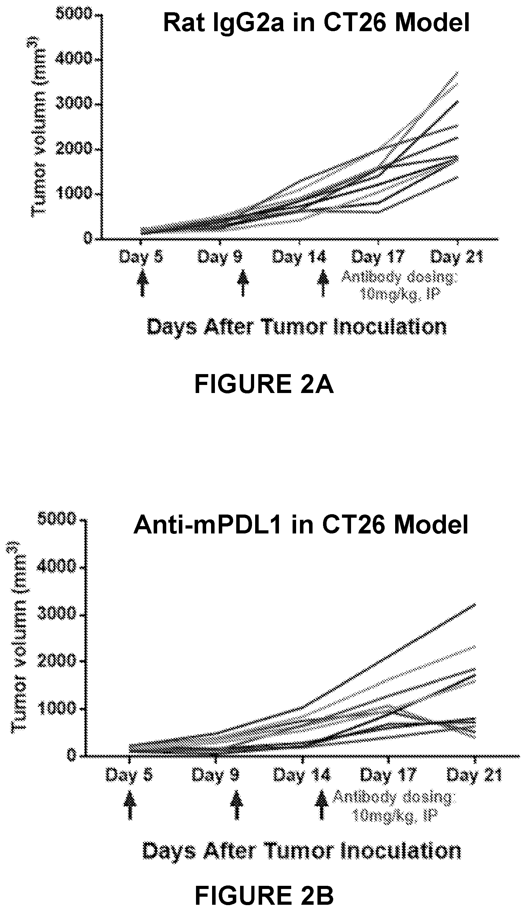

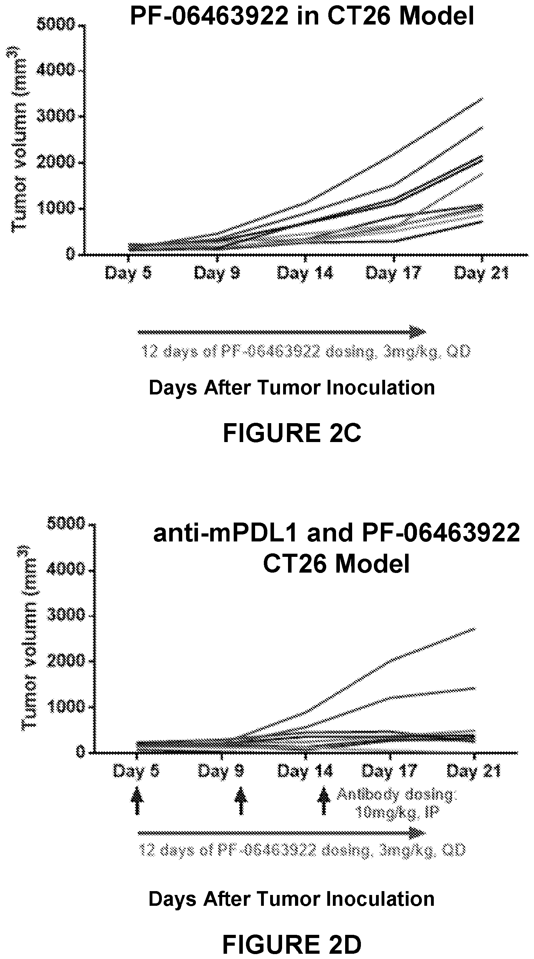

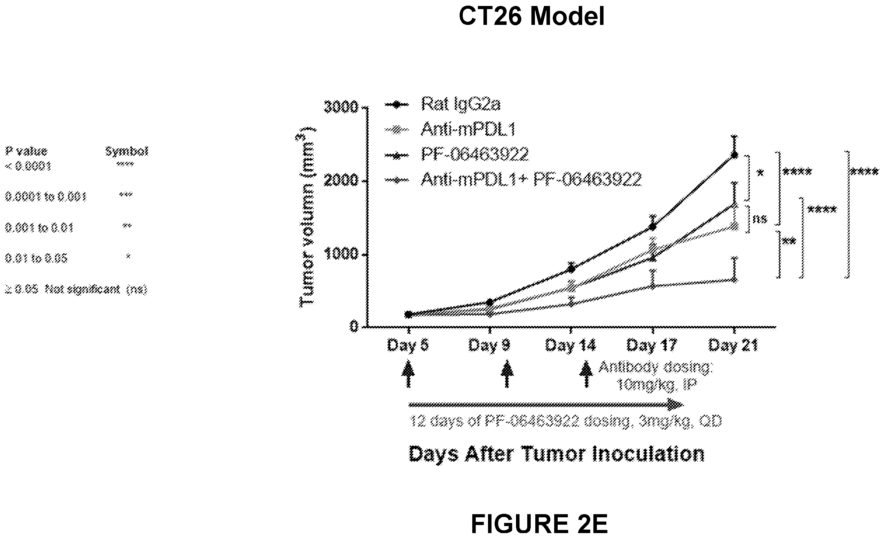

To evaluate the anti-tumor efficacy of combination immunotherapy against established CT26 and B16 tumors, anti-PD-L1 dosing regimens were selected based on prior studies that showed efficacy in the CT26 and B16 models (data not shown). Both CT26 colon carcinoma and B16 melonoma cell lines have published mutanome and transcriptome data, with no known ALK fusion events or oncogenic mutations. Castle et al, BMC Genomics, 15:190 (13 Mar 2014); Castle et al, CAN 11:3722 (11 Jan 2012). As shown in Figures 2A-2C, administration of an ALK inhibitor, an anti-PD-L1 antibody, or isotype alone did not consistently inhibit CT26 tumor growth when these single agent treatments were started in tumors of 100 - 150 mm³ in size. On day 21 post tumor inoculation, administration of each of an anti-PD-L1 antibody and an ALK inhibitor (PF-06463922) individually, resulted in 41.3% and 28.3% of tumor growth inhibition (TGI) respectively relative to the isotype control (p < 0.001). By contrast, as shown in Figures 2D and 2E, when animals were concurrently administrated an ALK inhibitor and an anti-PD-L1 antibody, a dramatic efficacy of 72% TGI, (2/10 animals were tumor free) relative to isotype controls (p < 0.0001) ensued. The percentage of TGI was defined as 1-(Tumor volumn_{Treated}/Tumor volumn_{Isotype control}) × 100.

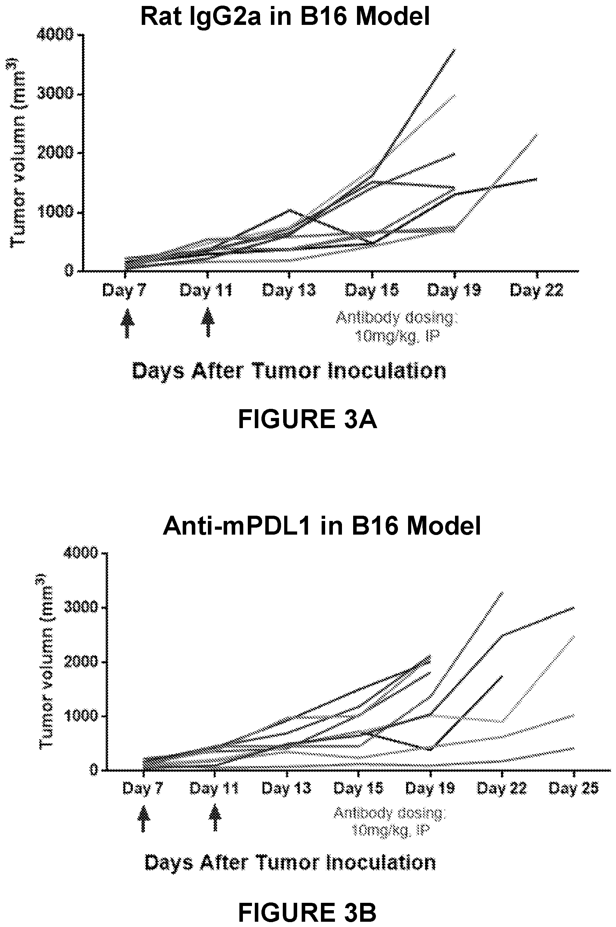

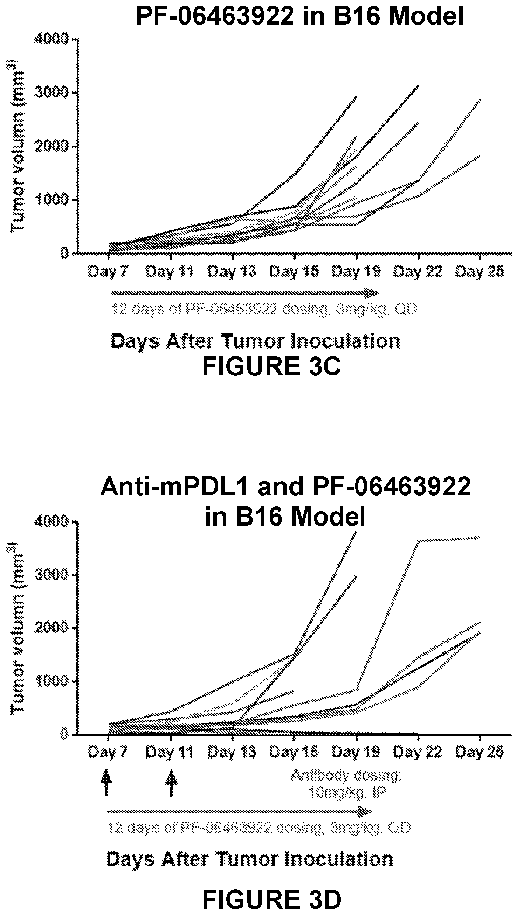

Similarly, an anti-tumor effect of the ALK inhibitor/anti-PD-L1 combination was observed in the B16 skin melanoma cancer model as shown in Figures 3A through 3D. As shown in Figure 3D, at the end of study (day 22 post tumor implant), 2 out of 10 animals had a complete response (tumor free), as compared to no animals having a complete response in those groups administered with a single agent isotype, ALK inhibitor or anti-mPD-L1 antibody.

### Materials and Methods

**Mice.** Six- to eight-week old female Balb/c mice were purchased from The Jackson Laboratories (Farmington, CT). Mice were maintained and all animal experiments were conducted according to the protocols approved by the Institutional Animal Care and Use Committee of Rinat, South San Francisco and Worldwide Research and Development (WRD), La Jolla, Pfizer Inc.

**Cell lines.** The CT26 colon carcinoma cell line was purchased from ATCC^{®} CRL-2638^{™}. B16-F10 ('B16') melonoma cell lines were purchased from ATCC^{®} CRL-6475^{™}. Cells were cultured in DMEM medium supplemented with 10% fetal bovine serum, and 100 I.U./mL penicillin/streptomycin at 37°C in an atmosphere of 5% CO₂ in air, and IMPACT-tested for pathogens at Research Animal Diagnostic Laboratory (RADIL) (Columbia, MO). Pathogen-free cells growing in an exponential growth phase were harvested and used for tumor inoculation.

**Antibodies for immunotherapy.** Rat anti-mouse PD-L1 mAb (clone MIH5) was purchased from eBioscience. Rat IgG2a isotype control were purchased from BioXcell (clone 2A3)(West Lebanon, NH).

**In vivo tumor efficacy studies.** Balb/c mice were inoculated subcutaneously at the right lower flank with 0.1 ml of 1×10⁶ CT26 murine colorectal cells in PBS. For the second study, C57BL/6 mice were inoculated subcutaneously in the right lower flank with 0.1 ml of 1×10⁶ B16 skin melanoma cells. When tumor bearing mice achieved a mean tumor volume of 100 to 150 mm³, mice were randomly assigned to 1 of 4 treatment groups, n= 10. Group 1 : received 10 mg/kg of an isotype control antibody (ratIgG2a, clone2A3) intraperitoneally every 3 days for a total of 3 doses; Group 2: received 10 mg/kg anti-PD-L1 antibody (MIH5) (ratIgG2a, purchased from eBioscience) intraperitoneally every 3 days for a total of 3 doses; Group 3: received 3 mg/kg of PF-06463922 an ALK ihibitor, orally, once daily for 12 days; Group 4: received 10 mg/kg each of an anti-PD-L1 antibody MIH5, LOTE20708-102, intraperitoneally every 3 days for a total of 3 doses plus PF-06463922 an ALK inhibitor orally, once daily for 12 days. Mice were monitored for tumor growth and body weight changes.

In the CT26 carcinoma model, blockade of PD-L1 with anti-PD-L1 antibody MIH5 or treatment with ALK inhibitor PF-06463922 was partially effective compared with isotype antibody treated group as a single agent therapy at preventing tumor growth (Figures 2B and 2C). Combination treatment with anti-PD-L1 antibody and ALK inhibitor significantly inhibited tumor growth and was significantly more effective than anti-PD-L1 antibody or ALK inhibitor treatment alone (Figures 2D, 2E). Furthermore, co-treatment at an early stage of tumor growth resulted not only in significant reduction of tumor volume but also demonstrated a sustained response. Early intervention resulted in about a 20% complete response (tumor free) and 50% partial response that were maintained for at least 21 days. These results indicate that ALK inhibition enhanced the anti-tumor activity of PD-L1 blockade and therefore worked synergistically with anti-PD-L1 antibodies to inhibit tumor growth (Figure 2E).

**Table 5: Average tumor volume (mm³)**

| Tumor vol (mm³) | Rat IgG2a | Anti-mPDL1 | PF-06463922 | Anti-mPDL1+ PF-06463922 |
|---|---|---|---|---|
| | Mean | Mean | Mean | Mean |
| Day 5 | 183.04 ±12.06385 | 182.29 ±12.52759 | 181.85 ±12.6828 | 179.65 ±14.20208 |
| Day 9 | 352.23 ±34.61456 | 263.34 ±46.57493 | 253.14 ±34.78116 | 185.5556 ±24.95714 |
| Day 14 | 806.38 ±80.18948 | 545.33 ±95.02131 | 548.44 ±96.03331 | 323.4 ±95.76349 |
| Day 17 | 1384.24 ±147.2062 | 1069.88 ±160.7312 | 962.02 ±180.1961 | 570.0889 ±215.698 |
| Day 21 | 2366.76 ±254.8813 | 1388.77 ±292.6458 | 1696.96 ±285.9975 | 662.9556 ±294.3596 |

Moreover, early intervention in the second B16 melonoma study, resulted in a 20% complete response (2/10 animals tumor free) maintained for 21 days that was not seen in any of the single agent groups as shown in Figure 3D.

### Example 3: ALK inhibition of crizotinib but not alectinib enhanced anti-tumor activity of anti-PD-L1 antibody in ALK-negative colorectal tumors in vivo

To evaluate the anti-tumor efficacy of ALK inhibitors crizotinib and alectinib in combination with immunotherapy against established CT26 and MC38 colon carcinoma cell lines, anti-PD-L1 dosing regimens were selected as in Example 2. CT26 colon carcinoma cell line has published mutanome and transcriptome data, with no known ALK fusion events or oncogenic mutations. Castle et al, BMC Genomics, 15:190 (13 Mar 2014); Castle et al, CAN 11:3722 (11 Jan 2012). In-house mRNA data analysis on murine syngeneic tumor cell lines and their in vivo derived tissues revealed that MC38, CT26, and B16F10 are negative in ALK and ROS1 mRNA level, while positive in cMET mRNA expression when compared to a house keeping gene.

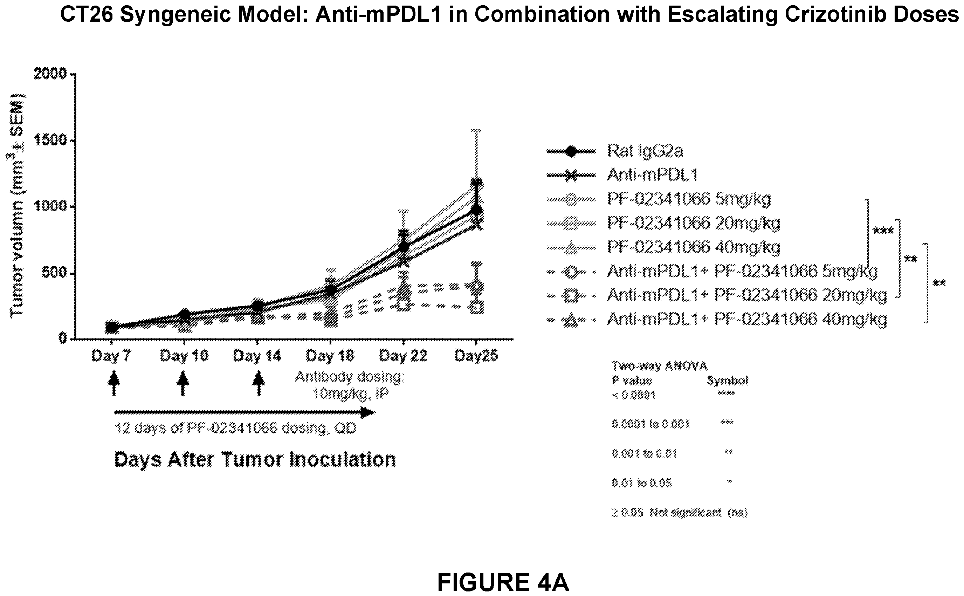

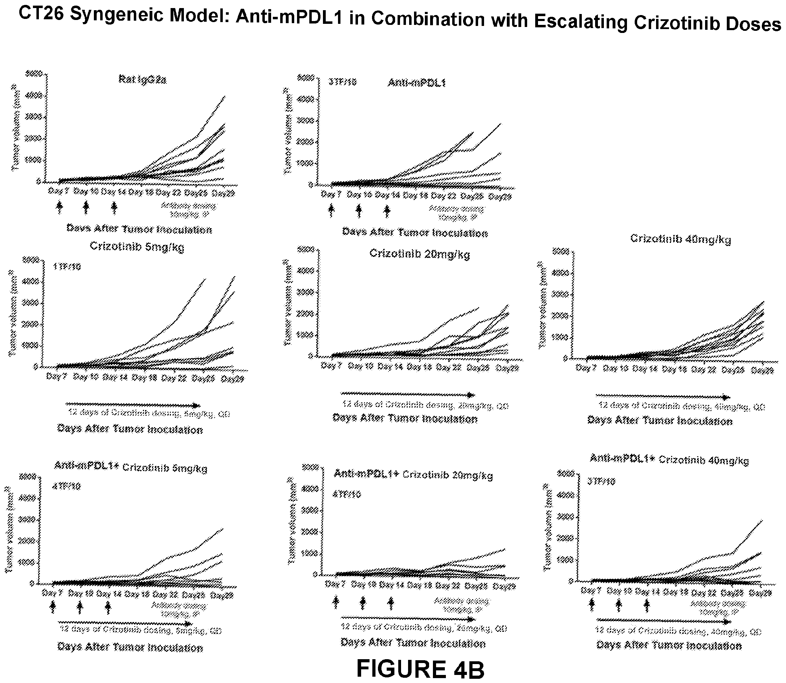

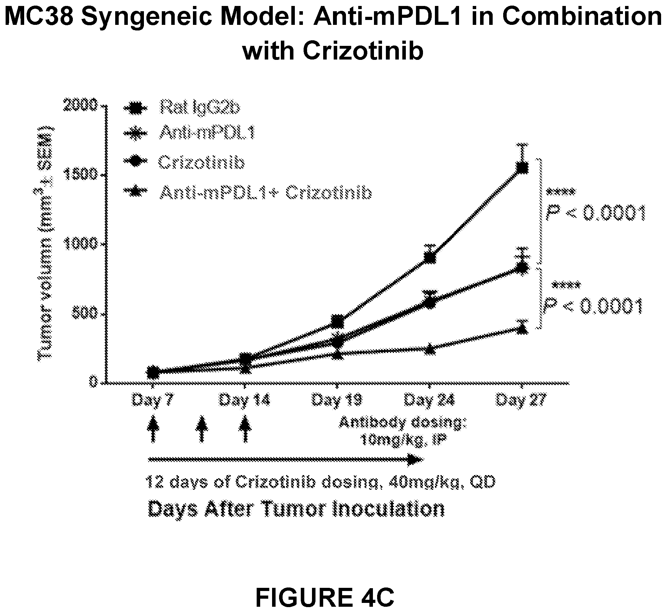

As shown in Figures 4A-4B, in the CT26 Syngeneic studies, no anti-tumor effects were observed from treatment with anti-mPD-L1 alone or with any dose of crizotinib alone compared to the isotype control antibody treated group. By Day 25, anti-mPD-L1 in combination with 5, 20, or 40 mg/kg crizotinib resulted in tumor growth inhibition (TGI) of 65.4% (P value = 0.0003) with 40% complete tumor regression (4 out 10 animals tumor free), 74.3% (P value < 0.0001) with 40% complete tumor regression (4 out 10 animals tumor free), or 61.3% (P value = 0.0004) with 30% complete tumor regression (3 out 10 animals tumor free), respectively (Figure 4A, 4B). Percentage of TGI was defined as (1- Tumor volumn_{Treated}/Tumor volumn_{Isotype control}) × 100%. Similarly, an enhanced anti-tumor effect of the ALk inhibitor crizotinib/anti-PD-L1 combination was observed in the MC38 colon carcinoma model. As shown in Figure 4C, by Day 24, anti-mPD-L1 in combination with crizotinib resulted in tumor growth inhibition (TGI) of 56.6% (P value = 0.0017) compared to the crizotinib alone and 57.4% (P value =0.0011) compared to the anti-mPDL1 treated group.

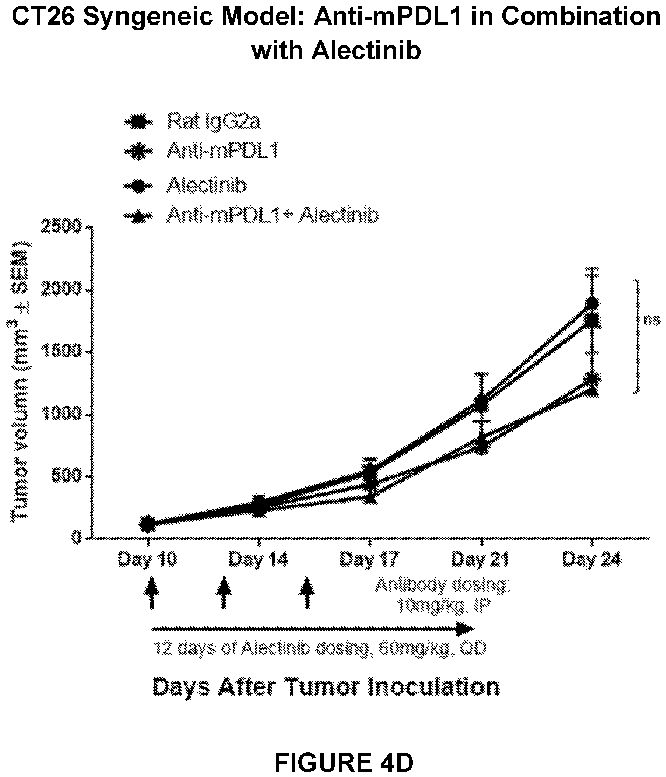

As shown in Figure 4D, ALK-inhibitor alectinib in combination with anti-mPDL1 treatment did not show significant improvement over either single agent alone. The lack of efficacy may result from any number of factors, including alectinib's selectivity for ALK and other kinases.

### Materials and Methods

**Mice.** Six- to eight-week old female Balb/c or C57BL/6 mice were purchased from The Jackson Laboratories. Mice were maintained and all animal experiments were conducted according to the protocols approved by the Institutional Animal Care and Use Committee of Rinat, South San Francisco and Worldwide Research and Development (WRD), La Jolla, Pfizer Inc.

**Materials.** CT26 cell lines, anti-PD-L1 antibody and Rat IgG isotypes (IgG2a and IgG2b) were purchased from BioXCell (clones 2A3 and LTF2, respectively) and prepared as described in Example 2 above.

**MC38 cell lines.** MC38 colon carcinoma cell line was kindly provided by Dr. Antoni Ribas at UCLA, California. Cells were cultured in DMEM medium supplemented with 10% fetal bovine serum, and 100 I.U./mL penicillin/streptomycin at 37°C in an atmosphere of 5% CO₂ in air, and IMPACT-tested for pathogens at Research Animal Diagnostic Laboratory (RADIL) (Columbia, MO). Pathogen-free cells growing in an exponential growth phase were harvested and used for tumor inoculation.

**In vivo tumor efficacy studies**. Balb/c mice were inoculated subcutaneously at the right lower flank with 0.1 ml of 0.1×10⁶ CT26 murine colorectal cells in PBS. For the MC38 study, C57BL/6 mice were inoculated subcutaneously in the right lower flank with 0.2 ×10⁶ MC38 colon carcinoma cells. When tumor bearing mice achieved a mean tumor volume of 50 to 150 mm³, mice were randomly assigned to 1 of 4 treatment groups, n= 10. Group 1 received 10 mg/kg of an isotype control antibody intraperitoneally every 3 days for a total of 3 doses; Group 2 received 10 mg/kg anti-PD-L1 antibody (MIH5) intraperitoneally every 3 days for a total of 3 doses; Group 3-5 received one of three doses of ALK inhibitor (in CT26, Group 3-5 received 5, 20, and 40 mg/kg of crizotinib or 60 mg/kg alectinib, while MC38 Group 3 received 40 mg/kg crizotinib) each orally, once daily for 12 days; Group 6 received 10 mg/kg each of an anti-PD-L1 antibody MIH5, intraperitoneally every 3 days for a total of 3 doses plus ALK inhibitor orally, once daily for 12 days (for CT26 cell line, 5, 20, and 40 mg/kg crizotinib or 60 mg/kg alectinib, and for MC38 cell line, 40 mg/kg crizotinib). Mice were monitored for tumor growth and body weight changes.

In the CT26 carcinoma model, blockade of PD-L1 with anti-PD-L1 antibody MIH5 or treatment with ALK inhibitors were not effective compared with isotype antibody treated group as a single agent therapy at inhibiting preventing tumor growth (Figures 2C, 4A, and 4B). In both CT26 and MC38 models, combination treatment with anti-PD-L1 antibody and crizotinib synergistically inhibited tumor growth and was significantly more effective than anti-PD-L1 antibody or crizotinib alone (Figures 4A, 4B and 4C). Furthermore, co-treatment at an early stage of tumor growth resulted not only in significant reduction of tumor volume but also demonstrated a sustained response. Early intervention resulted in about a 30-40% complete response (tumor free). These results indicate that ALK inhibition with crizotinib enhanced the anti-tumor activity of PD-L1 blockade and therefore worked synergistically with anti-PD-L1 antibodies to inhibit tumor growth (Figures 4A, 4B and 4C).

**Table 6. Tumor Measurement for Crizotinib Dose Escalation and Anti-mPDL1 Combination in CT26 Model Over Time**

| **Tumor Volume (mm3) (Mean ± SEM)** | | | | | | |
|---|---|---|---|---|---|---|
| **Days** | **Rat IgG2a** | **Anti-mPDL** | **Crizotinib (5mg/kg)** | **Crizotinib (20mg/kg)** | **Crizotinib (40mg/kg)** | |
| **7** | 89.6 ± 9.3 | 90.2 ± 8.3 | 90.3 ± 8.1 | 90.3 ± 8.0 | 94.4 ± 7.9 | |
| **10** | 189.4 ± 12.7 | 146.5 ± 19.1 | 140.9 ± 18.0 | 156.1 ± 21.9 | 127.0 ± 9.8 | |
| **14** | 253.2 ± 14.0 | 204.5 ± 32.3 | 246.6 ± 54.7 | 234.2 ± 49.0 | 218.5 ± 31.5 | |
| **18** | 374.1 ± 36.4 | 344.3 ± 106.7 | 407.1 ± 122.2 | 299.3 ± 67.1 | 319.6 ± 47.7 | |
| **22** | 698.1 ± 121.3 | 587.4 ± 205.7 | 749.8 ± 220.0 | 631.3 ± 158.5 | 685.3 ± 101.1 | |
| **25** | 979.4 ± 198.5 | 868.0 ± 333.5 | 1161.1 ± 415.4 | 934.9 ± 223.8 | 1074.5 ± 136.2 | |

| **Days** | **Anti-mPDL1+ Crizotinib (5mg/kg)** | **P value** | **Anti-mPDL1+ Crizotinib (20mg/kg)** | **P value** | **Anti-mPDL1+ Crizotinib (40mg/kg)** | **P value** |
|---|---|---|---|---|---|---|
| **7** | 90.2 ± 7.5 | ns | 89.8 ± 7.3 | ns | 89.7 ± 7.3 | ns |
| **10** | 110.7 ± 20.6 | ns | 110.9 ± 18.1 | ns | 103.5 ± 8.8 | ns |
| **14** | 164.9 ± 32.2 | ns | 183.7 ± 32.6 | ns | 166.7 ± 24.6 | ns |
| **18** | 163.4 ± 44.1 | ns | 148.8 ± 36.0 | ns | 200.4 ± 43.6 | ns |
| **22** | 346.6 ± 126.2 | ns | 264.6 ± 84.2 | ns | 402.3 ± 107.4 | ns |
| **25** | 402.0 ± 177.9 | 0.0003 | 239.9 ± 104.1 | < 0.0001 | 416.2 ± 148.3 | 0.000 4 |

Tumor volume (mm³) is shown as Mean ± SEM. P value is the comparison of anti-mPD-L1+ Crizotinib vs. Crizotnibi treated group. P < 0.05 is considered statistically significant, and "ns" denotes non-significant difference.

**Table 7. Tumor Measurements for Crizotinib and Anti-mPDL1 Combination in MC38 Model Over Time**

| **Tumor Volume (mm3) (Mean ± SEM)** | | | | | |
|---|---|---|---|---|---|
| **Days** | **Rat IgG2b** | **Anti-mPDL1** | **Crizotinib** | **Anti-mPDL1+ Crizotinib** | **P value** |
| **7** | 77.9 ± 5.4 175.3 ± | 78.2 ± 5.6 166.5 ± | 78.1 ± 6.0 169.7 ± | 78.1 ± 6.3 | ns |
| **14** | 12.8 | 11.5 | 16.3 | 111.2 ± 9.6 | ns |
| **19** | 439.5 ± 50.5 | 322.3 ± 28.7 | 291.7 ± 26.2 | 214.5 ± 20.6 | ns |
| **24** | 905.8 ± 89.9 | 590.8 ± 69.3 | 580.5 ± 67.0 | 251.8 ± 31.4 | 0.0017 |
| **27** | 1555 ± 166.4 | 830.4 ± 83.1 | 836.5 ± 137.8 | 401.7 ± 50.6 | <0.0001 |

Tumor volume (mm³) is shown as Mean ± SEM. P value is the comparison of Anti-mPDL1 + Crizotinib vs. Crizotinib treated group. P < 0.05 is considered statistically significant, and "ns" denotes non-significant difference.

By contrast, intervention with alectinib in the CT26 carcinoma study did not result in a significant improvement any of the single agent groups (Figure 4D).

**Table 8. Tumor Measurements for Alectinib and Anti-mPDL1 Combination in CT26 Model Over Time**

| **Tumor Volume (mm3) (Mean ± SEM)** | | | | | |
|---|---|---|---|---|---|
| **Days** | **Rat IgG2a** | **Anti-mPDL1** | **Alectinib** | **Anti-mPDL1+ Alectinib** | **P value** |
| **7** | 119.7 ± 11.6 | 119.1 ± 12.4 | 119.1 ± 12.5 | 119.2 ± 12.8 | ns |
| **14** | 268.2 ± 36.6 | 253.5 ± 59.8 | 293.4 ± 54.2 | 230.0 ± 40.3 | ns |
| **19** | 530.2 ± 103.5 | 438.4 ± 152.8 | 546.8 ± 98.0 | 337.7 ± 81.5 | ns |
| **24** | 1076.4 ± 247.5 | 738.9 ± 210.4 | 1114.2 ± 217.0 | 816.7 ± 247.7 | ns |
| **27** | 1759.9 ± 357.7 | 1285.9 ± 414.6 | 1892.5 ± 283.1 | 1202.4 ± 295.0 | ns |

Tumor volume (mm³) is shown as Mean ± SEM. P value is the comparison of Anti-mPDL1 + Alectinib vs. Alectinib treated group. P < 0.05 is considered statistically significant, and "ns" denotes non-significant difference.

### Example 4: CD8+ T cells is required for enhanced anti-tumor efficacy resulting from crizotinib in combination with anti-PD-L1 antibody treatment.

To evaluate the effect of crizotinib in combination with anti-PD-L1 on immune cells, *in vivo* depletion of CD4⁺, CD8⁺, or CD4⁺ concurrently with CD8⁺ T cells were performed prior to combination treatment in CT26 syngeneic model.

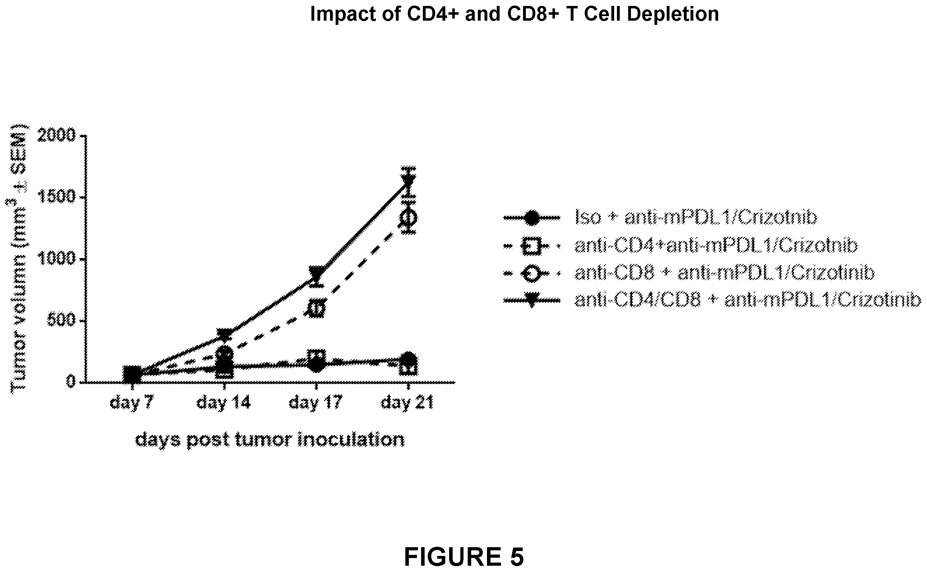

As shown in Figure 5, compared to isotype-depletion antibody treatment, *in vivo* depletion of CD4⁺ T cells did not affect the anti-tumor efficacy of the ALK/PD-L1 combination treated group. However *in vivo* depletion of CD8⁺ T cells alone, or the concurrent depletion of both CD4⁺ and CD8⁺T cells, led to loss of anti-tumor efficacy by the ALK/PD-L1 combination treatment group. By Day 21, as compared to the isotype depletion antibody-treated group (Group I), the anti-CD8⁺ depletion antibody group (Group 3) or the anti-CD4⁺ and anti-CD8⁺ concurrent depletion antibodies group (Group 4) resulted in tumor growth inhibition (TGI) of -92.95% (P value <0.0001) and -91.5% (P value <0.0001) respectively. Percentage of TGI was defined as (1- Tumor volumn_{depletion}/Tumor volumn_{Isotype control}) × 100%.

### Materials and Methods

**Mice.** Six- to eight-week old female Balb/c mice were purchased from The Jackson Laboratories. Mice were maintained and all animal experiments were conducted according to the protocols approved by the Institutional Animal Care and Use Committee of Rinat, South San Francisco and Worldwide Research and Development (WRD), La Jolla, Pfizer Inc.

**Materials.** CT26 cell lines, Rat IgG2b (clone LTF-2), anti-mouse CD4 (Rat IgG2b, GK1.5) and anti-mouse CD8 (Rat IgG2b, 2.43) were purchased from Bioexcel (West Lebanon, NH) and prepared as described in Example 2 above. As described above, rat anti-mouse PD-L1 mAb (clone MIH5) was purchased from eBioscience.

**In vivo tumor efficacy studies.** Balb/c mice were inoculated subcutaneously at the right lower flank with 0.1 ml of 0.1×10⁶ CT26 murine colorectal cells in PBS. When tumor bearing mice achieved a mean tumor volume of 50 to 150 mm³, mice were randomly assigned to 1 of 4 treatment groups, n= 10. Depletion antibodies were given intraperitoneally every 3 days and started one day prior to any drug treatment (crizotinib or PD-L1 or combo). Group 1 received 10 mg/kg of an isotype control depletion antibody (IgG2b); Group 2 received 10 mg/kg anti-CD4 depletion antibody (Rat IgG2b, GK1.5); Group 3 received 10 mg/kg anti-CD8 depletion antibody (Rat IgG2b, 2.43) and Group 4 was administered combination of anti-CD4 and anti-CD8 depletion antibodies. Crizotinib was dosed orally at 20 mg/kg, QD for 14 days. Murine surrogate anti-PD-L1 antibody (MIH5) was given intraperitoneally every 3 days for a total of 3 doses and started on concurrently with Crizotinib. Mice were monitored for tumor growth and body weight changes.

In the CT26 carcinoma model, in vivo depletion of CD4⁺ T cells does not affect the anti-tumor efficacy under combination treatment when compared to Isotype depletion antibody treated group. In vivo depletion of CD8⁺ T cells alone, or concurrent depletion of CD4⁺ and CD8⁺ T cells, leads to a significant loss of anti-tumor efficacy for the combination treatment group. By Day 21, compared to isotype depletion antibody treated group, anti-CD8⁺ depletion antibody or anti-CD4⁺ and anti-CD8⁺ depletion antibodies concurrently treated group results in tumor growth inhibition (TGI) of -92.95% (P value <0.0001) and -91.5% (P value <0.0001) respectively. These results indicate that the synergetic anti-tumor efficacy from ALK inhibition with crizotinib and PD-L1 blockade is indeed mediated through the regulation of immune cells.

**Table 9. Tumor Measurements for in vivo CD4⁺ and CD8⁺ T cell depletion with Crizotinib and Anti-mPDL1 Combination treatment in CT26 Model Over Time**

| **Days** | **Tumor Volume (mm3) (Mean ± SEM)** | | | | **P value** |
|---|---|---|---|---|---|
| | **Rat IgG2b depletion** | **CD4⁺ Depletion** | **CD8⁺ Depletion** | **CD4⁺ plus CD8⁺ Depletion** | |
| **7** | 66.4 ± 4.5 | 65.9 ± 4.4 | 65.8 ± 4.3 | 65.4 ± 4.4 | ns |
| **14** | 133.3 ± 16.5 | 106.8 ± 17.2 | 232.8 ± 25.0 | 378.0 + 20.5 | ns |
| **17** | 147.9 ± 20.9 | 196.0 ± 60.9 | 606.3 ± 64.4 | 860.4 ± 73.3 | < 0.0001 |
| **21** | 190.6 ± 26.1 | 138.2 ± 62.4 | 1342.4 ± 120.6 | 1626.5 ± 115.8 | < 0.0001 |

| | | | | | |
|---|---|---|---|---|---|
| Tumor volume (mm³) is shown as Mean ± SEM. P value is the comparison of CD8+ depletion vs. Rat IgG2b treated group. P < 0.05 is considered statistically significant, and "ns" denotes non-significant difference. | | | | | |

### Example 5: ALK inhibition with crizotinib enhanced anti-tumor activity of avelumab in ALK-negative colorectal tumors in vivo

To evaluate the anti-tumor efficacy of combination immunotherapy against established MC38 tumors, avelumab dosing regimens were selected as 20 mg/kg per mouse intraperitoneally (i.p.) for total 3 doses and 3-4 days apart.

This study was conducted in MC38 colon carcinoma model, where the animals were treated with crizotinib at 40mg/kg, QD for 12 days combined with fixed avelumab dose. Avelumab is a fully human IgG1 antibody that recognizes both human and mouse PD-L1. Administration of avelumab alone resulted in 49.7% TGI (P value <0.0001), crizotinib alone resulted in 48.5% TGI (P value <0.0001), and the combination of avelumab with crizotinib led to 65% TGI (P value <0.0001) compared with the isotype control antibody treated group (Table 10 below). No statistically significant effect on body weight change was observed in animals under all treatment conditions.

### Materials and Methods

**Mice.** Six- to eight-week old female C57BL/6 mice were purchased from The Jackson Laboratories (Farmington, CT). Mice were maintained and all animal experiments were conducted according to the protocols approved by the Institutional Animal Care and Use Committee of Rinat, South San Francisco and Worldwide Research and Development (WRD), La Jolla, Pfizer Inc.

**Cell lines.** MC38 colon carcinoma cell line was kindly provided by Dr. Antoni Ribas at UCLA, California. Cells were cultured in DMEM medium supplemented with 10% fetal bovine serum, and 100 I.U./mL penicillin/streptomycin at 37°C in an atmosphere of 5% CO₂ in air, and IMPACT-tested for pathogens at Research Animal Diagnostic Laboratory (RADIL) (Columbia, MO). Pathogen-free cells growing in an exponential growth phase were harvested and used for tumor inoculation.

**Antibodies for immunotherapy.** Avelumab mAb (human IgG1) was provided by Merck Serono at 20 mg/mL concentration, Lot:508203. Human IgG1 was prepared in-house (Lot No. NB123249p192CA110124). Antibody was prepared at concentration of 1mg/mL in phosphate buffered saline (PBS) (Invitrogen, Carlsbad, CA), and dosed 0.2 mL per mouse intraperitoneally (i.p.) for 3 doses 3-4 days apart.

**In vivo tumor efficacy studies.** C57BL/6 mice were used for subcutaneous tumor inoculation with 2× 10⁵ MC38 cells in 0.1 mL of PBS. When tumors reached an average of 100 mm³, mice were randomized into groups of 10 animals per group, and treatment was started on the same day. Tumor size was measured in two dimensions using a digital caliper, and the volume was expressed in mm³ using the formula: V= 0.5 L ×W² where L and W are the long and short diameters of the tumor, respectively. Body weight was recorded weekly. Percentage of TGI was defined as 1- (Tumor volume _{Treated}/ Tumor volume _{Isotype control}) × 100. Treatment Group 1 received 20 mg/kg of an isotype control antibody (humanIgG1) intraperitoneally every 4 days for a total of 3 doses; Group 2 received 20 mg/kg avelumab intraperitoneally every 4 days for a total of 3 doses; Group 3 received 40 mg/kg of crizotinib, orally, once daily for 12 days; Group 4 received 20 mg/kg avelumab intraperitoneally every 4 days for a total of 3 doses plus 40 mg/kg crizotinib orally, once daily for 12 days. Mice were monitored for tumor growth and body weight changes.

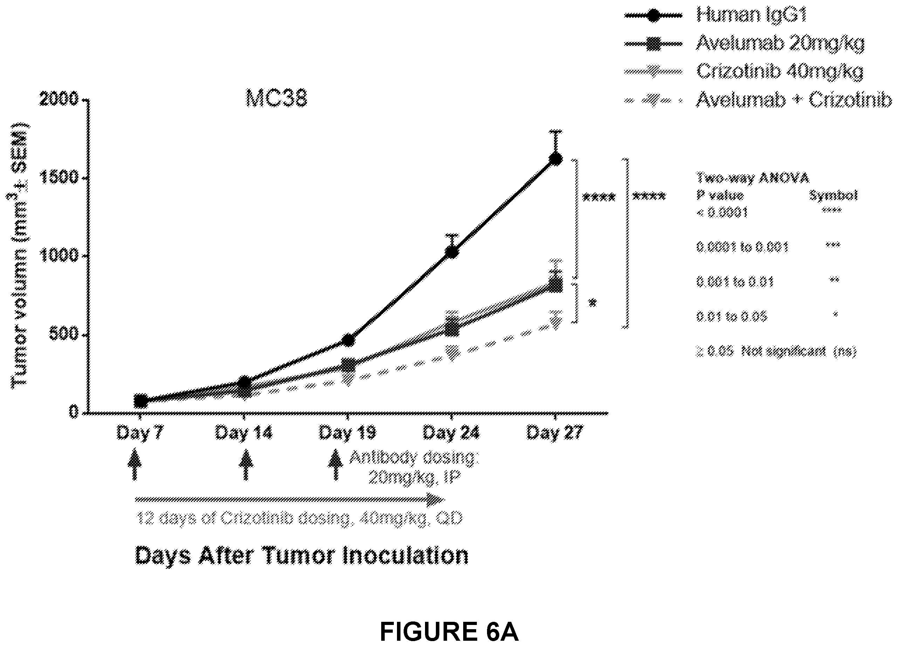

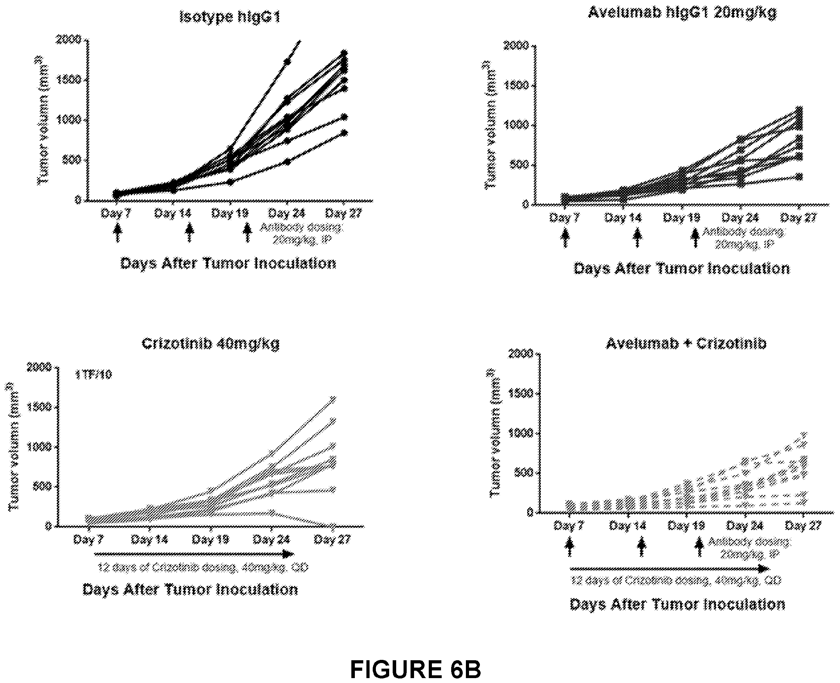

As shown in Figures 6A and 6B, a significant anti-tumor effect of the crizotinib/avelumab combination was observed in the MC38 colon carcinoma model.

**Table 10. Tumor Measurements for MC38 Model Over Time**

| **Days** | **Tumor Volume (mm3) (Mean ± SEM)** | | | | **P value** |
|---|---|---|---|---|---|
| | **Human IgG1** | **Avelumab** | **Crizotinib** | **Avelumab+ Crizotinib** | |
| **7** | 78.2 ± 5.1 | 78.3 ± 5.4 | 78.1 ± 6.0 | 77.8 ± 6.5 | ns |
| **14** | 199.6 ± 10.0 | 145.5 ± 11.0 | 169.7 ± 16.3 | 116.0 ± 11.1 | ns |
| **19** | 465.4 ± 35.7 | 307.1 ± 25.5 | 291.7 ± 26.2 | 210.8 ± 30.7 | 0.0405 |
| **24** | 1030.1 ± 106.0 | 535.0 ± 62.0 | 580.5 ± 67.0 | 367.0 ± 56.3 | <0.0001 |
| **27** | 1625.0 ± 174.4 | 817.7 ± 87.2 | 836.5 ± 137.8 | 568.0 ± 81.6 | <0.0001 |

| | | | | | |
|---|---|---|---|---|---|
| Tumor volume (mm³) is shown as Mean ± SEM. P value is the comparison of Avelumab + Crizotinib vs. isotype control antibody (human IgG1) treated group. P < 0.05 is considered statistically significant, and "ns" denotes non-significant difference. | | | | | |

### Example 6: Studies to evaluate efficacy and safety of the ALK inhibitors, crizotinib and lorlatinib (PF06463922), in combination with PD-L1 inhibitor, avelumab

The ALk inhibitors, crizotinib and lorlatinib, and PD-L1 inhibitor, avelumab, in combination can be studied in an open-label dose finding study to evaluate safety, efficacy, pharmacokinetics and pharmacodynamics in patients with either ALK-positive or ALK-negative locally advanced or metastatic NSCLC. The study can begin with a screening period of up to and including 28 days prior to the first dose of study drugs to assess eligibility. The treatment period begins on the first day of the first cycle (14 days), see combination schedule in Tables A and B below, and lasts for 2 cycles.

Treatment with ALK inhibitors, crizotinib or lorlatinib, with avelumab may for example continue until the patient experiences unacceptable toxicity that precludes further treatment and/or disease progression.

The study can consist of dose-finding and dose-expansion phase.

### 1. Dose-finding phase

Both ALK-positive and ALK-negative groups will be evaluated to identify the maximum tolerated dose (MTD) and recommended Phase 2 dose (RP2D). Determination of the MTD will be performed using modified toxicity probability index (mTPI) wth dose deescalation from the approved prescribed dose of crizotinib (250 mg BID), 100 mg QD of lorlatinib and the RP2D of avelumab (10 mg/kg Q2W). In the ALK-positive group (Group B), a dose expansion will evaluate an additional 12 patients at the MTD/RP2D to further assess safety, pharmacokinetics, pharmacodynamics and antitumor activity of the combination.

**Table A. Crizotinib plus Avelumab Dose Levels (Group A) in patients with ALK-negative NSCLC**

| | **Avelumab** | |
|---|---|---|
| **Crizotinib** | **5 mg/kg IV** | **10 mg/kg IV** |
| **250 mg BID PO** | DL-1A* | DL 0 (Start) |
| **200 mg BID PO** | DL-1A-1C | DL-1C** |
| **250 mg QD PO** | DL-1A-2C | DL-2C** |

| | | |
|---|---|---|
| *-1A denotes dose reduction attributed to avelumab (A=avelumab) **-1C denotes dose reduction attributed to crizotinib (C=crizotinib), -2C denotes dose reduction attributed to crizotinib (C=crizotinib). | | |

**Table B. Lorlatinib plus Avelumab Dose Levels (Group B) in patients with ALK-positive NSCLC**

| | **Avelumab** | |
|---|---|---|
| **Lorlatinib** | **5 mg/kg IV** | **10 mg/kg IV** |
| **100 mg QD PO** | DL-1A* | DL-0 (Start) |
| **75 mg QD PO** | DL-1A*-1P** | DL-1P** |
| **50 mg QD PO** | DL-1A*-2P** | DL-2P** |

| | | |
|---|---|---|
| *-1A denotes dose reduction attributed to avelumab (A=avelumab) **-1P, -2P denotes dose reduction attributed to lorlatinib, PF-06463922 (P=PF-06463922) | | |

The MTD estimate is the highest dose tested of crizotinib and avelumab or lorlatinib and avelumab associated with the occurrence of DLTs within the first 2 cycles of treatment in <33% of patients. The RP2D is the dose of crizotinib and avelumab or lorlatinib and avelumab in combination chosen for further clinical development. If the MTD proves to be clinically feasible for long-term administration in a reasonable number of patients, this dose may become the RP2D. Further experience in Phase 1b may result in a RP2D dose lower than the MTD.

### 2. Expansion phase

In Group B, a dose expansion will evaluate an additional 12 patients at the MTD/RP2D to further assess the safety, pharmacokinetics, pharmacodynamics and antitumor activity of the combination of lorlatinib with avelumab in ALK-positive patients with NSCLC.

### Phase 2 (Group A only)

After the MTD is identified and the RP2D is determined in Group A, the 12 patients treated at the RP2D will be considered Stage 1 of Simon's Optimal Two-Stage design. If 3 or more patients of the 12 in Phase 1b have a confirmed response per RECIST 1.1, then in Phase 2 an additional 33 patients will be enrolled and treated. If there are fewer than 3 patients who have a confirmed objective response in the first 12 patients treated at the RP2D, then Phase 2 will not be opened for enrollment. Patients with ALK-negative NSCLC who are enrolled and subsequently determined by retrospective central testing to be positive for ALK gene rearrangement, ROS1 gene translocation, c-Met gene amplification, or c-Met exon 14 deletion may be replaced.

### Dose-expansion

In the ALK-positive group (Group B, above), a dose expansion will evaluate an additional 12 patients at the MTD/RP2D to further assess the safety, pharmacokinetics, pharmacodynamics and antitumor activity of the combination.

In the ALK-negative group (Group A, above), after the MTD is identified and the RP2D is determined, the 12 patients treated at the RP2D will be considered Stage 1 of Simon's Optimal Two-Stage design. If 3 or more patients of the 12 in Phase 1b have a confirmed response per RECIST 1.1, then in Phase 2 an additional 33 patients will be enrolled and treated. If there are fewer than 3 patients who have a confirmed objective response in the first 12 patients treated at the RP2D, then Phase 2 will not be opened for enrollment. Patients with ALK-negative NSCLC who are enrolled and subsequently determined by retrospective central testing to be positive for ALK gene rearrangement, ROS1 gene translocation, c-Met gene amplification, or c-Met exon 14 deletion may be replaced. Patients with NSCLC containing epidermal growth factor receptor (EGFR) mutations are permitted into the ALk-negative group if they have exhausted appropriate targeted therapy for these mutations.

The data cut-off for the primary clinical study report can occur once all patients in the expansion phase have completed treatment or have discontinued earlier.

### Example 7: Study to evaluate efficacy and safety of ALK-inhibitor, crizotinib in combination with PD-L1 inhibitor, avelumab, in patients with ALK-negative NSLC

Group A patients were enrolled on the basis of locally approved testing for ALK-negative NSCLC tumor biopsy, subject to confirmation with retrospective central testing. Four patients from Group A had data available for Investigator Assessment: 3 patients with Overall Response as Progressive Disease and 1 patient with unconfirmed Stable Disease. These data are preliminary and have not yet been verified against source documents. The amount of time between first dose and follow-up scan at Investigator Assessment was approximately 8 weeks, per protocol. The clinical study from which this preliminary data was generated specifies RECIST v1.1 for evaluation of response.

## Claims

1. An anti-PD-L1 antibody for use in combination with crizotinib, or a pharmaceutically acceptable salt or solvate thereof, for treating or delaying progression of an ALK-negative cancer in an individual.

2. The anti PD-L1 antibody for use according to Claim 1, which antibody is selected from the group consisting of: MPDL3280A, MDX-1105, MEDI4736 and MSB0010718C.

3. The anti-PD-L1 antibody for use according to claim 1 or 2, wherein the ALK-negative cancer is selected from the group consisting of melanoma, colon cancer, bladder cancer, breast cancer, clear cell kidney cancer, head/neck squamous cell carcinoma, rectal cancer, lung squamous cell carcinoma, thyroid cancer, bladder cancer, cervical cancer, uterine cancer, endometrial cancer, lung adenocarcinoma, ovarian cancer, papillary kidney cancer, non-small-cell lung cancer (NSCLC), ovarian cancer, pancreatic cancer, prostate cancer, renal cell cancer, small-cell lung cancer (SCLC) and triple negative breast cancer.

4. The anti-PD-L1 antibody for use according to any of claims 1-3, wherein the crizotinib, or pharmaceutically acceptable salt or solvate thereof, is administered before the PD-1 axis binding antagonist, simultaneous with the anti-PD-L1 antibody or after the anti-PD-L1 antibody.

5. The anti-PD-L1 antibody for use according to any one of claims 1-4, wherein the individual has an ALK-negative cancer selected from the group consisting of colorectal cancer, melanoma, non-small cell lung cancer, ovarian cancer, breast cancer, pancreatic cancer, hematological malignancy, renal cell carcinoma.

6. An anti-PD-L1 antibody for use in combination with crizotinib, or a pharmaceutically acceptable salt or solvate thereof, for enhancing immune function in an individual having an ALk-negative cancer.

7. A medicament comprising an anti-PD-L1 antibody, for use in combination with crizotinib, or a pharmaceutically acceptable salt or solvate thereof, for treating ALK-negative cancer in an individual.

8. A medicament comprising crizotinib, or a pharmaceutically acceptable salt or solvate thereof, for use in combination with an anti-PD-L1 antibody for treating ALK-negative cancer in an individual.

9. The anti-PD-L1 antibody for use according to claims 1-8, wherein the anti-PD-L1 antibody is administered intravenously, intramuscularly, subcutaneously, topically, orally, transdermally, intraperitoneally, intraorbitally, by implantation, by inhalation, intrathecally, intraventricularly, or intranasally.

10. The anti-PD-L1 antibody for use according to any of the preceding claims, wherein the ALK-negative cancer does not overexpress c-Met and/or ROS1 and/or have no known alterations in either c-Met or Ros1.

## Patentansprüche

1. Anti-PD-L1-Antikörper zur Verwendung in Kombination mit Crizotinib oder einem pharmazeutisch unbedenklichen Salz oder Solvat davon zur Behandlung oder Verzögerung der Progression einer ALK-negativen Krebserkrankung bei einem Individuum.

2. Anti-PD-L1-Antikörper zur Verwendung nach Anspruch 1, wobei der Antikörper ausgewählt ist aus der Gruppe bestehend aus: MPDL3280A, MDX-1105, MED14736 und MSB0010718C.

3. Anti-PD-L1-Antikörper zur Verwendung nach Anspruch 1 oder 2, wobei die ALK-negative Krebserkrankung ausgewählt ist aus der Gruppe bestehend aus Melanom, Darmkrebs, Blasenkrebs, Brustkrebs, klarzelligem Nierenkrebs, Plattenepithelkarzinom des Kopfes/Halses, Rektumkarzinom, Plattenepithelkarzinom der Lunge, Schilddrüsenkrebs, Blasenkrebs, Zervixkarzinom, Uteruskarzinom, Endometriumkarzinom, Adenokarzinom der Lunge, Ovarialkarzinom, papillärem Nierenkarzinom, nichtkleinzelligem Lungenkrebs (NSCLC), Ovarialkarzinom, Bauchspeicheldrüsenkrebs, Prostatakrebs, Nierenzellkarzinom, nichtkleinzelligem Lungenkrebs (SCLC) und tripelnegativem Brustkrebs.

4. Anti-PD-L1-Antikörper zur Verwendung nach einem der Ansprüche 1-3, wobei das Crizotinib oder pharmazeutisch unbedenkliche Salz oder Solvat vor dem PD-1-Achse bindenden Antagonisten, gleichzeitig mit dem Anti-PD-L1-Antikörper oder nach dem Anti-PD-L1-Antikörper verabreicht wird.

5. Anti-PD-L1-Antikörper zur Verwendung nach einem der Ansprüche 1-4, wobei das Individuum eine ALK-negative Krebserkrankung ausgewählt aus der Gruppe bestehend aus Kolorektalkarzinom, Melanom, nichtkleinzelligem Lungenkrebs, Ovarialkarzinom, Brustkrebs, Bauchspeicheldrüsenkrebs, hämatologischem Malignom, Nierenzellkarzinom aufweist.

6. Anti-PD-L1-Antikörper zur Verwendung in Kombination mit Crizotinib oder einem pharmazeutisch unbedenklichen Salz oder Solvat davon, zur Verbesserung der Immunfunktion bei einem an einer ALk-negativen Krebserkrankung leidenden Individuum.

7. Arzneimittel, umfassend einen Anti-PD-L1-Antikörper, zur Verwendung in Kombination mit Crizotinib oder einem pharmazeutisch unbedenklichen Salz oder Solvat davon, zur Behandlung einer ALK-negativen Krebserkrankung bei einem Individuum.

8. Arzneimittel, umfassend Crizotinib oder ein pharmazeutisch unbedenkliches Salz oder Solvat davon, zur Verwendung in Kombination mit einem Anti-PD-L1-Antikörper, zur Behandlung einer ALK-negativen Krebserkrankung bei einem Individuum.

9. Anti-PD-L1-Antikörper zur Verwendung nach einem der Ansprüche 1-8, wobei der Anti-PD-L1-Antikörper intravenös, intramuskulär, subkutan, topisch, oral, transdermal, intraperitoneal, intraorbital, über Implantation, über Inhalation, intrathekal, intraventrikulär oder intranasal verabreicht wird.

10. Anti-PD-L1-Antikörper zur Verwendung nach einem der vorhergehenden Ansprüche, wobei bei der ALKnegativen Krebserkrankung keine Überexpression von c-Met und/oder ROS1 erfolgt und/oder weder in c-Met noch in Ros1 bekannte Veränderungen vorliegen.

## Revendications

1. Anticorps anti-PD-L1 pour une utilisation en combinaison avec le crizotinib, ou un sel ou solvate pharmaceutiquement acceptable correspondant, pour le traitement ou le retardement de la progression d'un cancer ALK-négatif chez un individu.

2. Anticorps anti-PD-L1 pour une utilisation selon la revendication 1, lequel anticorps est choisi dans le groupe constitué par MPDL3280A, MDX-1105, MEDI4736 et MSB0010718C.

3. Anticorps anti-PD-L1 pour une utilisation selon la revendication 1 ou 2, le cancer ALK-négatif étant choisi dans le groupe constitué par un mélanome, un cancer du côlon, un cancer de la vessie, un cancer du sein, un cancer du rein à cellules claires, un carcinome à cellules squameuses de la tête/du cou, un cancer rectal, un carcinome à cellules squameuses du poumon, un cancer de la thyroïde, un cancer de la vessie, un cancer du col de l'utérus, un cancer utérin, un cancer de l'endomètre, un adénocarcinome du poumon, un cancer de l'ovaire, un cancer du rein papillaire, un cancer du poumon non à petites cellules (NSCLC), un cancer de l'ovaire, un cancer pancréatique, un cancer de la prostate, un cancer de cellules rénales, un cancer du poumon à petites cellules (SCLC) et un cancer du sein triplement négatif.

4. Anticorps anti-PD-L1 pour une utilisation selon l'une quelconque des revendications 1 à 3, le crizotinib, ou un sel ou solvate pharmaceutiquement acceptable correspondant, étant administré avant l'antagoniste se liant à l'axe PD-1, simultanément avec l'anticorps anti-PD-L1 ou après l'anticorps anti-PD-L1.

5. Anticorps anti-PD-L1 pour une utilisation selon l'une quelconque des revendications 1 à 4, l'individu étant atteint d'un cancer ALK-négatif choisi dans le groupe constitué par un cancer colorectal, un mélanome, un cancer du poumon non à petites cellules, un cancer de l'ovaire, un cancer du sein, un cancer pancréatique, une malignité hématologique, un carcinome de cellules rénales.

6. Anticorps anti-PD-L1 pour une utilisation en combinaison avec le crizotinib, ou un sel ou solvate pharmaceutiquement acceptable correspondant, pour l'amélioration de la fonction immunitaire chez un individu atteint d'un cancer ALk-négatif.

7. Médicament comprenant un anticorps anti-PD-L1, pour une utilisation en combinaison avec le crizotinib, ou un sel ou solvate pharmaceutiquement acceptable correspondant, pour le traitement d'un cancer ALK-négatif chez un individu.

8. Médicament comprenant du crizotinib, ou un sel ou solvate pharmaceutiquement acceptable correspondant, pour une utilisation en combinaison avec un anticorps anti-PD-L1 pour le traitement d'un cancer ALK-négatif chez un individu.

9. Anticorps anti-PD-L1 pour une utilisation selon l'une quelconque des revendications 1 à 8, l'anticorps anti-PD-L1 étant administré de manière intraveineuse, de manière intramusculaire, de manière sous-cutanée, de manière topique, de manière orale, de manière transdermique, de manière intrapéritonéale, de manière intraorbitale, par implantation, par inhalation, de manière intrathécale, de manière intraventriculaire ou de manière intranasale.

10. Anticorps anti-PD-L1 pour une utilisation selon l'une quelconque des revendications précédentes, le cancer ALK-négatif ne surexprimant pas c-Met et/ou ROSI et/ou n'ayant pas d'altérations connues dans c-Met ou Ros1.
